# EUROPEAN PATENT APPLICATION

(11) **EP 2 327 734 A1**
(43) Date of publication of application: **01.06.2011**
(21) Application number: 09811497.8
(22) Date of filing: 26.08.2009
(51) Int. Cl.: C08G 61/12, C07D 417/14, C08L 65/00, C09K 11/06, H01L 29/786, H01L 51/05, H01L 51/30, H01L 51/42, H01L 51/50

(54) **POLYMER COMPOUND AND POLYMER LIGHT-EMITTING ELEMENT UTILIZING SAME**

(30) Priority: 03.09.2008 JP 2008225707
(71) Applicant: Sumitomo Chemical Company, Limited, Tokyo 104-8260 (JP); Sumation Co., Ltd., Chuo-ku Tokyo 104-8260 (JP)
(72) Inventor: MORIWAKI, Shota, Tsukuba-shi Ibaraki 305-0821 (JP); GOTO, Osamu, Tsukuba-shi Ibaraki 305-0821 (JP); ASADA, Kohei, Tsukuba-shi Ibaraki 305-0821 (JP)
(74) Representative: Duckworth, Timothy John
(86) International application number: PCT/JP2009/065285
(87) International publication number: WO 2010/026972

(57) **Abstract**

A polymer compound comprising a repeating unit represented by the formula (I): 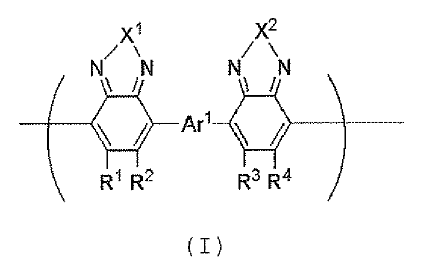 [wherein X¹ and X² are the same or mutually different and represent an oxygen atom, a sulfur atom, -N(R^{N})- or C(R^{c1})=C(R^{c2})-, R¹, R², R³, R⁴, R^{N}, R^{c1} and R^{c2} are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and Ar¹ represents a di-valent heterocyclic group.].

## Description

### Technical Field

The present invention relates to a polymer compound and a polymer light emitting device using the same.

### Background Art

As materials used in polymer light emitting devices, various polymer compounds are investigated, and a polymer compound containing as a repeating unit a fluorenediyl group and a di-valent group represented by the following formula is known as the example thereof (see, Japanese Patent Application National Publication (Laid-Open) No. 2004-534863).

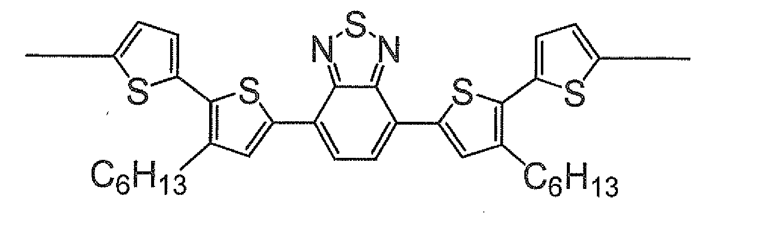

### Disclosure of the Invention

However, a polymer light emitting device fabricated by using the above-described polymer compound is not sufficient yet in the lifetime of luminance from emission initiation point until 10% reduction (hereinafter, referred to as "luminance 10% reduction lifetime").

The present invention has an object of providing a polymer compound affording a long luminance 10% reduction lifetime when used in a polymer light emitting device.

In a first aspect, the present invention provides a polymer compound comprising a repeating unit represented by the formula (I): 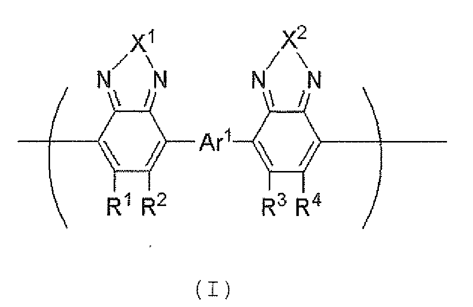 [wherein X¹ and X² are the same or mutually different and represent an oxygen atom, a sulfur atom, -N(R^{N})- or C(R^{c1})=C(R^{c2})-, R¹, R², R³, R⁴, R^{N}, R^{c1} and R^{c2} are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and Ar¹ represents a di-valent heterocyclic group.].

In a second aspect, the present invention provides a compound represented by the formula (VI): 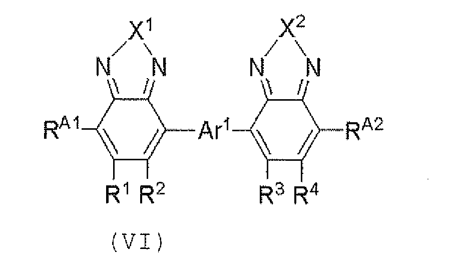 [wherein X¹ and X² are the same or mutually different and represent an oxygen atom, a sulfur atom, -N(R^{N})- or C(R^{c1})=C(R^{C2})-, R¹, R², R³, R⁴, R^{N}, R^{A1}, R^{A2}, R^{c1} and R^{c2} are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and Ar¹ represents a di-valent heterocyclic group.].

In a third aspect, the present invention provides a method of producing a polymer compound having a repeating unit represented by the formula (III) described later, comprising condensation-polymerizing a compound represented by the formula (VIII) described later in the presence of a transition metal compound.

In a fourth aspect, the present invention provides a composition comprising the above-described polymer compound.

In a fifth aspect, the present invention provides a film comprising the above-described polymer compound.

In a sixth aspect, the present invention provides a polymer light emitting device having an anode, a cathode, and an organic layer containing the above-described polymer compound disposed between the anode and the cathode.

In a seventh aspect, the present invention provides an organic transistor having a source electrode, a drain electrode, a gate electrode, and an organic layer containing the above-described polymer compound.

In an eighth aspect, the present invention provides a photoelectric conversion device having an anode, a cathode, and an organic layer containing the above-described polymer compound disposed between the anode and the cathode.

### Modes for Carrying Out the Invention

The polymer compound of the present invention comprises a repeating unit represented by the formula (I). In the formula (I), X¹ and X² are the same or mutually different and represent an oxygen atom, a sulfur atoms, -N(R^{N})- or C(R^{c1})=C(R^{c2})-, R¹, R², R³, R⁴, R^{N}, R^{c1} and R^{c2} are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and Ar¹ represents a di-valent heterocyclic group.

The halogen atom includes a fluorine atom, a chlorine atom, a bromine atom and an iodine atom.

The alkyl group may be linear or branched, and may also be a cycloalkyl group. The alkyl group has a carbon atom number of usually 1 to 20. Examples of the alkyl group include a methyl group, an ethyl group, a n-propyl group, an i-propyl group, a n-butyl group, an i-butyl group, a t-butyl group, a s-butyl group, a 3-methylbutyl group, a n-pentyl group, a n-hexyl group, a 2-ethylhexyl group, a n-heptyl group, a n-octyl group, a n-nonyl group, a n-decyl group, a 3,7-dimethyloctyl group and a n-lauryl group. A hydrogen atom in the above-described alkyl group is optionally substituted by a fluorine atom. The alkyl group substituted by a fluorine atom includes a trifluoromethyl group, a pentafluoroethyl group, a perfluorobutyl group, a perfluorohexyl group, a perfluorooctyl group and the like.

The alkoxy group may be linear or branched, and may also be a cycloalkyloxy group. The alkoxy group has a carbon atom number of usually 1 to 20. Examples of the alkoxy group include a methoxy group, an ethoxy group, a n-propyloxy group, an i-propyloxy group, a n-butoxy group, an i-butoxy group, a s-butoxy group, a t-butoxy group, a n-pentyloxy group, a n-hexyloxy group, a cyclohexyloxy group, a n-heptyloxy group, a n-octyloxy group, a 2-ethylhexyloxy group, a n-nonyloxy group, a n-decyloxy group, a 3,7-dimethyloctyloxy group and a n-lauryloxy group. A hydrogen atom in the above-described alkoxy group is optionally substituted by a fluorine atom. The alkoxy group substituted by a fluorine atom includes a trifluoromethoxy group, a pentafluoroethoxy group, a perfluorobutoxy group, a perfluorohexyloxy group, a perfluorooctyloxy group and the like.

The alkylthio group may be linear or branched, and may also be a cycloalkylthio group. The alkylthio group has a carbon atom number of usually 1 to 20. Examples of the alkylthio group include a methylthio group, an ethylthio group, a n-propylthio group, an isopropylthio group, a n-butylthio group, an isobutylthio group, a s-butylthio group, a t-butylthio group, a n-pentylthio group, a n-hexylthio group, a cyclohexylthio group, a n-heptylthio group, a n-octylthio group, a 2-ethylhexylthio group, a n-nonylthio group, a n-decylthio group, a 3,7-dimethyloctylthio group and a n-laurylthio group. A hydrogen atom in the above-described alkylthio group is optionally substituted by a fluorine atom. The alkylthio group substituted by a fluorine atom includes a trifluoromethylthio group and the like.

The aryl group means an atomic group obtained by removing one hydrogen atom from an aromatic hydrocarbon, and includes also those having a condensed ring, and those having two or more independent benzene rings or condensed rings or two or more of both the rings, bonded directly or via a vinylene group or the like. The aryl group has a carbon atom number of usually 6 to 60, preferably 6 to 48. The above-described aryl group optionally has a substituent. This substituent includes linear or branched alkyl groups having a carbon atom number of 1 to 20, cycloalkyl groups having a carbon atom number of 3 to 20, alkoxy groups containing the alkyl group or the cycloalkyl group in its structure, groups represented by the formula (5), and the like. Examples of the aryl group include a phenyl group, C₁ to C₁₂ alkoxyphenyl groups (C₁ to C₁₂ means that the carbon atom number thereof is 1 to 12; the same shall apply hereinafter), C₁ to C₁₂ alkylphenyl groups, a 1-naphthyl group, a 2-naphthyl group, a 1-anthracenyl group, a 2-anthracenyl group, a 9-anthracenyl group, a pentafluorophenyl group and the like, and preferable are C₁ to C₁₂ alkoxyphenyl groups and C₁ to C₁₂ alkylphenyl groups. The C₁ to C₁₂ alkoxyphenyl group includes a methoxyphenyl group, an ethoxyphenyl group, a n-propyloxyphenyl group, an isopropyloxyphenyl group, a n-butoxyphenyl group, an isobutoxyphenyl group, a s-butoxyphenyl group, a t-butoxyphenyl group, a n-pentyloxyphenyl group, a n-hexyloxyphenyl group, a cyclohexyloxyphenyl group, a n-heptyloxyphenyl group, a n-octyloxyphenyl group, a 2-ethylhexyloxyphenyl group, a n-nonyloxyphenyl groups, a n-decyloxyphenyl group, a 3,7-dimethyloctyloxyphenyl group and a n-lauryloxyphenyl group. The C₁ to C₁₂ alkylphenyl group includes a methylphenyl group, an ethylphenyl group, a dimethylphenyl group, a n-propylphenyl group, a mesityl group, a methylethylphenyl group, an isopropylphenyl group, a n-butylphenyl group, an isobutylphenyl group, a s-butylphenyl group, a t-butylphenyl group, a n-pentylphenyl group, an isoamylphenyl group, a n-hexylphenyl group, a n-heptylphenyl group, a n-octylphenyl group, a n-nonylphenyl group, a n-decylphenyl group, a n-dodecylphenyl group, and the like. A hydrogen atom in the above-described aryl group is optionally substituted by a fluorine atom.

-O-(CH₂)_{g1}-O-(CH₂)ₕ₁-CH₃ (5)

(wherein g1 represents an integer of 1 to 6, and h1 represents an integer of 0 to 5.).

The aryloxy group has a carbon atom number of usually 6 to 60, preferably 6 to 48. Examples of the aryloxy group include a phenoxy group, C₁ to C₁₂ alkoxyphenoxy groups, C₁ to C₁₂ alkylphenoxy groups, a 1-naphthyloxy group, a 2-naphthyloxy group, a pentafluorophenyloxy group and the like, and preferable are C₁ to C₁₂ alkoxyphenoxy groups and C₁ to C₁₂ alkylphenoxy groups. The C₁ to C₁₂ alkoxy includes methoxy, ethoxy, n-propyloxy, isopropyloxy, n-butoxy, isobutoxy, s-butoxy, t-butoxy, n-pentyloxy, n-hexyloxy, cyclohexyloxy, n-heptyloxy, n-octyloxy, 2-ethylhexyloxy, n-nonyloxy, n-decyloxy, 3,7-dimethyloctyloxy and n-lauryloxy. The C₁ to C₁₂ alkylphenoxy group includes a methylphenoxy group, an ethylphenoxy group, a dimethylphenoxy group, a n-propylphenoxy group, a 1,3,5-trimethylphenoxy group, a methylethylphenoxy group, an isopropylphenoxy group, a n-butylphenoxy group, an isobutylphenoxy group, a s-butylphenoxy group, a t-butylphenoxy group, a n-pentylphenoxy group, an isoamylphenoxy group, a n-hexylphenoxy group, a n-heptylphenoxy group, a n-octylphenoxy group, a n-nonylphenoxy group, a n-decylphenoxy group, a n-dodecylphenoxy group and the like.

The arylthio group optionally has a substituent on an aromatic ring, and has a carbon atom number of usually 6 to 60. The arylthio group includes a phenylthio group, C₁ to C₁₂ alkoxyphenylthio groups, C₁ to C₁₂ alkylphenylthio groups, a 1-naphthylthio group, a 2-naphthylthio group, a pentafluorophenylthio group, a pyridylthio group, a pyridazinylthio group, a pyrimidylthio group, a pyrazylthio group and a triazylthio group.

The arylalkyl group optionally has a substituent, and has a carbon atom number of usually 7 to 60. The arylalkyl group includes phenyl C₁ to C₁₂ alkyl groups, C₁ to C₁₂ alkoxyphenyl C₁ to C₁₂ alkyl groups, C₁ to C₁₂ alkylphenyl C₁ to C₁₂ alkyl groups, 1-naphthyl C₁ to C₁₂ alkyl groups and 2-naphthyl C₁ to C₁₂ alkyl groups.

The arylalkoxy group optionally has a substituent, and has a carbon atom number of usually 7 to 60. The arylalkoxy group includes phenyl C₁ to C₁₂ alkoxy groups, C₁ to C₁₂ alkoxyphenyl C₁ to C₁₂ alkoxy groups, C₁ to C₁₂ alkylphenyl C₁ to C₁₂ alkoxy groups, 1-naphthyl C₁ to C₁₂ alkoxy groups and 2-naphthyl C₁ to C₁₂ alkoxy groups.

The arylalkylthio group optionally has a substituent, and has a carbon atom number of usually 7 to 60. The arylalkylthio group includes phenyl C₁ to C₁₂ alkylthio groups, C₁ to C₁₂ alkoxyphenyl C₁ to C₁₂ alkylthio groups, C₁ to C₁₂ alkylphenyl C₁ to C₁₂ alkylthio groups, 1-naphthyl C₁ to C₁₂ alkylthio groups and 2-naphthyl C₁ to C₁₂ alkylthio groups.

The arylalkenyl group has a carbon atom number of usually 8 to 60. Examples of the arylalkenyl group include phenyl C₂ to C₁₂ alkenyl groups, C₁ to C₁₂ alkoxyphenyl C₂ to C₁₂ alkenyl groups, C₁ to C₁₂ alkylphenyl C₂ to C₁₂ alkenyl groups, 1-naphthyl C₂ to C₁₂ alkenyl groups and 2-naphthyl C₂ to C₁₂ alkenyl groups, and preferable are C₁ to C₁₂ alkoxyphenyl C₂ to C₁₂ alkenyl groups and C₁ to C₁₂ alkylphenyl C₂ to C₁₂ alkenyl groups.

The arylalkynyl group has a carbon atom number of usually 8 to 60. Examples of the arylalkynyl group include phenyl C₂ to C₁₂ alkynyl groups, C₁ to C₁₂ alkoxyphenyl C₂ to C₁₂ alkynyl groups, C₁ to C₁₂ alkylphenyl C₂ to C₁₂ alkynyl groups, 1-naphthyl C₂ to C₁₂ alkynyl groups and 2-naphthyl C₂ to C₁₂ alkynyl groups, and preferable are C₁₂ to C₁₂ alkoxyphenyl C₂ to C₁₂ alkynyl groups and C₁ to C₁₂ alkylphenyl C₂ to C₁₂ alkynyl groups.

The mono-valent heterocyclic group means an atomic group remaining after removing one hydrogen atom from a heterocyclic compound. The mono-valent heterocyclic group has a carbon atom number of usually 4 to 60, preferably 4 to 20. The carbon atom number of the mono-valent heterocyclic group does not include the carbon atom number of the substituent. The above-described heterocyclic compound means an organic compound having a cyclic structure in which elements constituting the ring include not only a carbon atom, but also a hetero atom such as oxygen, sulfur, nitrogen, phosphorus, boron, silicon and the like contained in the ring. Examples of the mono-valent heterocyclic group include a thienyl group, C₁ to C₁₂ alkylthienyl groups, a pyrrolyl group, a furyl group, a pyridyl group, C₁ to C₁₂ alkylpyridyl groups, a piperidyl group, a quinolyl group and an isoquinolyl group, and preferable are a thienyl group, C₁ to C₁₂ alkylthienyl groups, a pyridyl group and C₁ to C₁₂ alkylpyridyl groups. Of the mono-valent heterocyclic groups, mono-valent aromatic heterocyclic groups are preferable.

The heterocyclic thio group means a group obtained by substituting a hydrogen atom of a mercapto group by a mono-valent heterocyclic group. The heterocyclic thio group includes hetero arylthio groups such as a pyridylthio group, a pyridazinylthio group, a pyrimidylthio group, a pyrazinylthio group, a triazinylthio group and the like.

The substituted amino group includes amino groups substituted by one or two groups selected from the group consisting of an alkyl group, an aryl group, an arylalkyl group and a mono-valent heterocyclic group, and the alkyl group, aryl group, arylalkyl group and mono-valent heterocyclic group optionally have a substituent. The carbon atom number of the substituted amino group is usually 1 to 60, preferably 2 to 48, not including the carbon atom number of the substituent. Examples of the substituted amino group include a methylamino group, a dimethylamino group, an ethylamino group, a diethylamino group, a n-propylamino group, a di-n-propylamino group, an isopropylamino group, a diisopropylamino group, a n-butylamino group, a s-butylamino group, an isobutylamino group, a t-butylamino group, a n-pentylamino group, a n-hexylamino group, a cyclohexylamino group, a n-heptylamino group, a n-octylamino group, a 2-ethylhexylamino group, a n-nonylamino group, a n-decylamino group, a 3,7-dimethyloctylamino group, a n-laurylamino group, a cyclopentylamino group, a dicyclopentylamino group, a cyclohexylamino group, a dicyclohexylamino group, a pyrrolidyl group, a piperidyl group, a ditrifluoromethylamino group, a phenylamino group, a diphenylamino group, C₁ to C₁₂ alkoxyphenylamino groups, di(C₁ to C₁₂ alkoxyphenyl) amino groups, di(C₁ to C₁₂ alkylphenyl) amino groups, a 1-naphthylamino group, a 2-naphthylamino group, a pentafluorophenylamino group, a pyridylamino group, a pyridazinylamino group, a pyrimidylamino group, a pyrazylamino group, a triazylamino group, phenyl C₁ to C₁₂ alkylamino groups, C₁ to C₁₂ alkoxyphenyl C₁ to C₁₂ alkylamino groups, C₁ to C₁₂ alkylphenyl C₁ to C₁₂ alkylamino groups, di(C₁ to C₁₂ alkoxyphenyl C₁ to C₁₂ alkyl) amino groups, di(C₁ to C₁₂ alkylphenyl C₁ to C₁₂ alkyl) amino groups, 1-naphthyl C₁ to C₁₂ alkylamino groups and 2-naphthyl C₁ to C₁₂ alkylamino groups.

The substituted silyl group includes silyl groups substituted by one, two or three groups selected from the group consisting of an alkyl group, an aryl group, an arylalkyl group and a mono-valent heterocyclic group. The substituted silyl group has a carbon atom number of usually 1 to 60, preferably 3 to 48. The alkyl group, aryl group, arylalkyl group and mono-valent heterocyclic group optionally have a substituent.

Examples of the substituted silyl group include a trimethylsilyl group, a triethylsilyl group, a tri-n-propylsilyl group, a tri-isopropylsilyl group, a dimethyl-isopropylsilyl group, a diethyl-isopropylsilyl group, a t-butyldimethylsilyl group, a n-pentyldimethylsilyl group, a n-hexyldimethylsilyl group, a n-heptyldimethylsilyl group, a n-octyldimethylsilyl group, a 2-ethylhexyl-dimethylsilyl group, a n-nonyldimethylsilyl group, a n-decyldimethylsilyl group, a 3,7-dimethyloctyl-dimethylsilyl group, a n-lauryldimethylsilyl group, phenyl C₁ to C₁₂ alkylsilyl groups, C₁ to C₁₂ alkoxyphenyl C₁ to C₁₂ alkylsilyl groups, C₁ to C₁₂ alkylphenyl C₁ to C₁₂ alkylsilyl groups, 1-naphthyl C₁ to C₁₂ alkylsilyl groups, 2-naphthyl C₁ to C₁₂ alkylsilyl groups, phenyl C₁ to C₁₂ alkyldimethylsilyl groups, a triphenylsilyl group, a tri-p-xylylsilyl group, a tribenzylsilyl group, a diphenylmethylsilyl group, a t-butyldiphenylsilyl group and a dimethylphenylsilyl group.

The acyl group has a carbon atom number of usually 2 to 20, preferably 2 to 18. Examples of the acyl group include an acetyl group, a propionyl group, a butyryl group, an isobutyryl group, a pivaloyl group, a benzoyl group, a trifluoroacetyl group and a pentafluorobenzoyl group.

The acyloxy group has a carbon atom number of usually 2 to 20, preferably 2 to 18. Examples of the acyloxy group include an acetoxy group, a propionyloxy group, a butyryloxy group, an isobutyryloxy group, a pivaloyloxy group, a benzoyloxy group, a trifluoroacetyloxy group and a pentafluorobenzoyloxy group.

The imine residue includes residues obtained by removing one hydrogen atom from imine compounds (namely, meaning organic compounds having -N=C- in the molecule. Examples thereof include aldimines, ketimines, and compounds obtained by substituting a hydrogen atom on N in these compounds by an alkyl group or the like.). The imine residue has a carbon atom number of usually 2 to 20, preferably 2 to 18. Examples of the imine residue include groups represented by the following structural formulae. 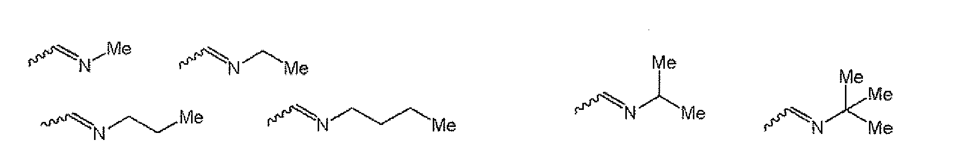 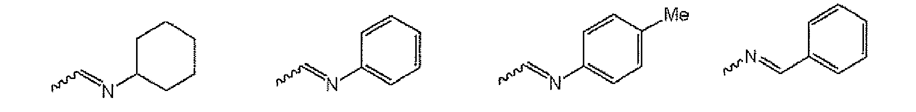 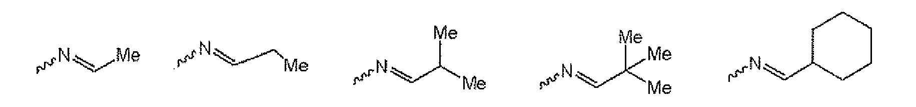 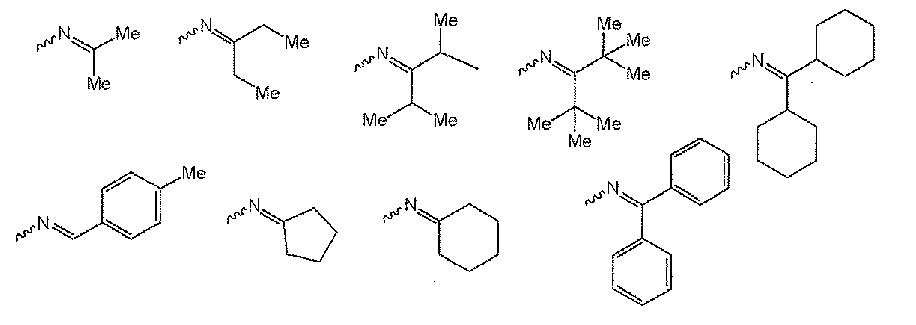 (wherein Me represents a methyl group, and the same shall apply hereinafter. The wavy line represents a connecting bond, and means a possibility of a geometric isomer such as a cis body, a trans body or the like depending on the kind of the imine residue.).

The amide group has a carbon atom number of usually 2 to 20, preferably 2 to 18. Examples of the amide group include a formamide group, an acetamide group, a propioamide group, a butyroamide group, a benzamide group, a trifluoroacetamide group, a pentafluorobenzamide group, a diformamide group, a diacetamide group, a dipropioamide group, a dibutyroamide group, a dibenzamide group, a ditrifluoroacetamide group and a dipentafluorobenzamide group.

The acid imide group includes residues obtained by removing from an acid imide one hydrogen atom connected to its nitrogen atom, and has a carbon atom number preferably of 4 to 20. Examples of the acid imide group include groups shown below.

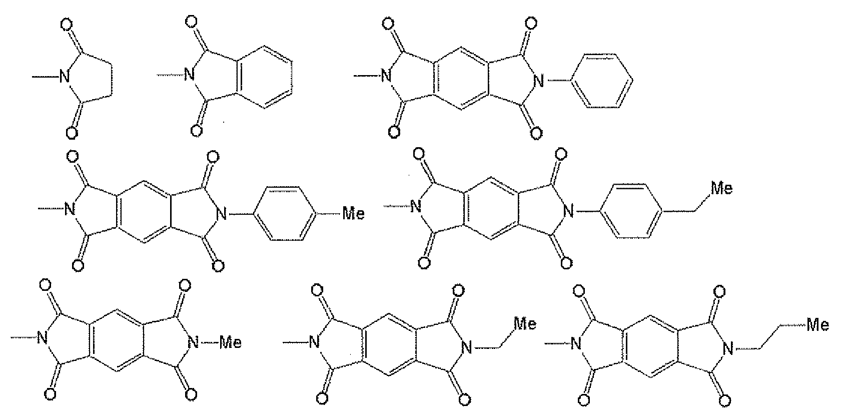

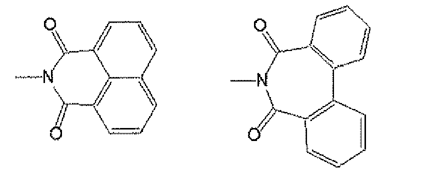

The substituted carboxyl group includes carboxyl groups substituted by an alkyl group, an aryl group, an arylalkyl group or a mono-valent heterocyclic group. The above-described alkyl group, aryl group, arylalkyl group and mono-valent heterocyclic group optionally have a substituent. The substituted carboxyl group has a carbon atom number of usually 2 to 60, preferably 2 to 48. The carbon atom number of the substituted carboxyl group does not include the carbon atom number of the substituent. Examples of the substituted carboxyl group include a methoxycarbonyl group, an ethoxycarbonyl group, a n-propoxycarbonyl group, an isopropoxycarbonyl group, a n-butoxycarbonyl group, an isobutoxycarbonyl group, a t-butoxycarbonyl group, a n-pentyloxycarbonyl group, a n-hexyloxycarbonyl group, a cyclohexyloxycarbonyl group, a n-heptyloxycarbonyl group, a n-octyloxycarbonyl group, a 2-ethylhexyloxycarbonyl group, a n-nonyloxycarbonyl group, a n-decyloxycarbonyl group, a 3,7-dimethyloctyloxycarbonyl group, a n-dodecyloxycarbonyl group, a trifluoromethoxycarbonyl group, a pentafluoroethoxycarbonyl group, a perfluorobutoxycarbonyl group, a perfluorohexyloxycarbonyl group, a perfluorooctyloxycarbonyl group, a phenoxycarbonyl group, a naphthoxycarbonyl group and a pyridyloxycarbonyl group.

From the standpoint of easiness of synthesis of a monomer, R¹, R², R³ and R⁴ in the (I) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom.

From the standpoint of lowering the emission starting voltage when the polymer compound of the present invention is used in a polymer light emitting device, X¹ and X² in the (I) represent preferably a sulfur atom or N(R^{N})-, more preferably a sulfur atom.

In the (I), Ar¹ represents a di-valent heterocyclic group.

The di-valent heterocyclic group means an atomic group remaining after removal of two hydrogen atoms from a heterocyclic compound, and this group optionally has a substituent.

The heterocyclic compound means an organic compound having a cyclic structure in which elements constituting the ring include not only a carbon atom, but also a hetero atom such as oxygen, sulfur, nitrogen, phosphorus, boron, arsenic and the like contained in the ring. Among di-valent heterocyclic groups, di-valent aromatic heterocyclic groups are preferable. From the standpoint of solubility, fluorescence property, easiness of synthesis, properties when made into a device, and the like, the substituent is preferably an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a mono-valent heterocyclic group, a carboxyl group, a substituted carboxyl group, a cyano group or a nitro group.

A portion of the di-valent heterocyclic group excluding the substituent has a carbon atom number of usually 3 to 60. The total carbon atom number of the di-valent heterocyclic group including the substituent is usually 3 to 100.

The di-valent heterocyclic group includes, for example, the following groups.

Di-valent heterocyclic groups containing a nitrogen atom as a hetero atom: pyridinediyl groups (the following formulae 101 to 106), diazaphenylene groups (the following formulae 107 to 110), quinolinediyl groups (the following formulae 111 to 125), quinoxalinediyl groups (the following formulae 126 to 130), acridinediyl groups (the following formulae 131 to 134), bipyridyldiyl groups (the following formulae 135 to 137), phenanthrolinediyl groups (the following formulae 138 to 140).

5-membered ring heterocyclic groups containing an oxygen atom, a silicon atom, a nitrogen atom, a sulfur atom, a selenium atom, a boron atom, a phosphorus atom or the like as a hetero atom (the following formulae 141 to 145).

5-membered ring condensed hetero groups containing an oxygen atom, a silicon atom, a nitrogen atom, a selenium atom or the like as a hetero atom (the following formulae 146 to 157).

5-membered ring heterocyclic groups containing an oxygen atom, a silicon atom, a nitrogen atom, a sulfur atom, a selenium atom or the like as a hetero atom, which are connected at an α-position of its hetero atom to form a dimer or an oligomer (the following formulae 158 to 159).

5-membered ring heterocyclic groups containing an oxygen atom, a silicon atom, a nitrogen atom, a sulfur atom, a selenium atom or the like as a hetero atom, which are connected at an α-position of its hetero atom to a phenyl group (the following formulae 160 to 166).

5-membered ring condensed heterocyclic groups containing an oxygen atom, a nitrogen atom, a sulfur atom, a selenium atom or the like as a hetero atom, and carrying thereon a substituent such as a phenyl group, a furyl group or a thienyl group (the following formulae 167 to 172).

Groups containing an oxygen atom, a silicon atom, a nitrogen atom, a sulfur atom, a selenium atom or the like as a hetero atom, and having a fluorene-like structure (the following formulae 173 to 202).

6-membered ring condensed hetero groups containing an oxygen atom, a silicon atom, a nitrogen atom, a sulfur atom, a selenium atom, a boron atom, a phosphorus atom or the like as a hetero atom (the following formulae 203 to 205).

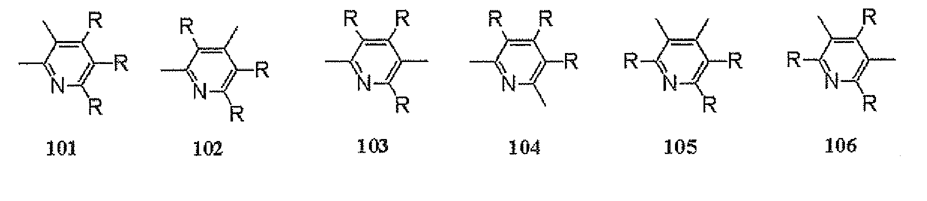

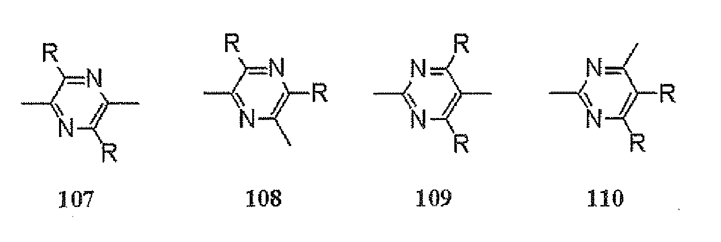

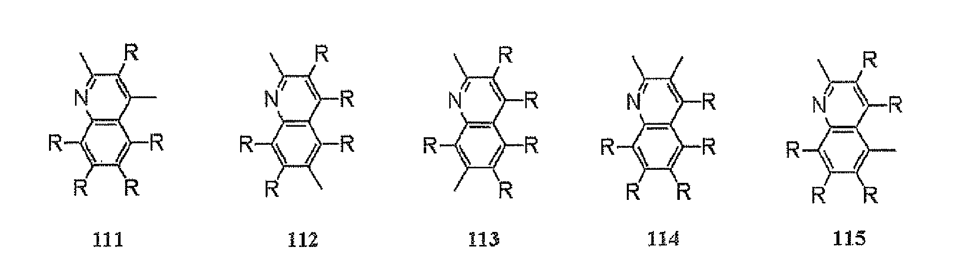

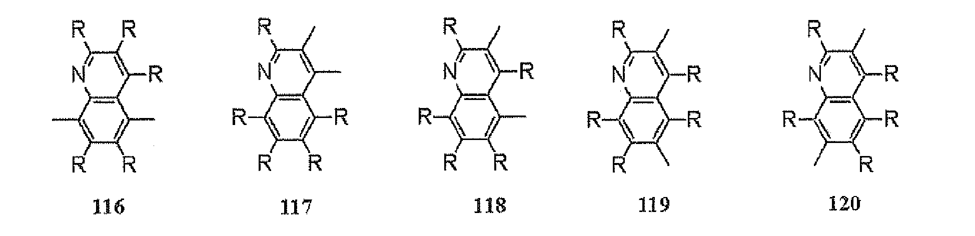

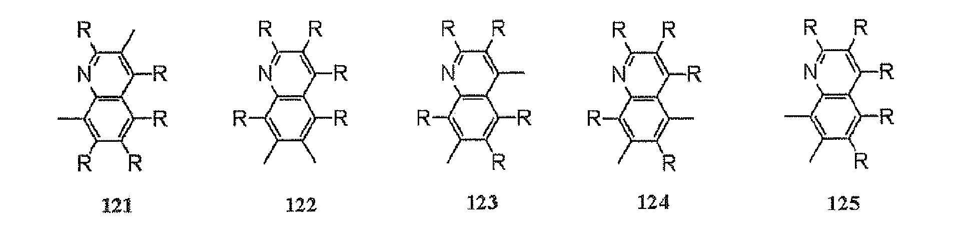

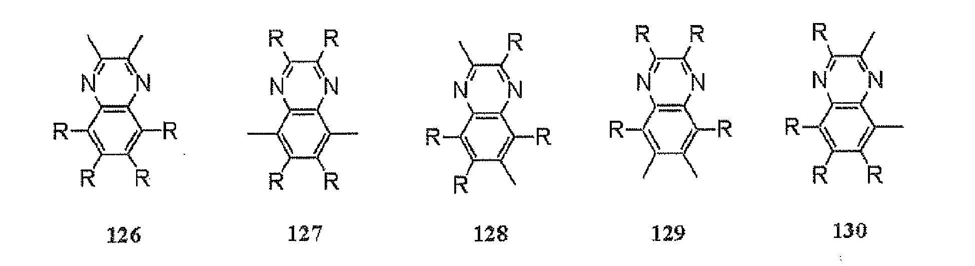

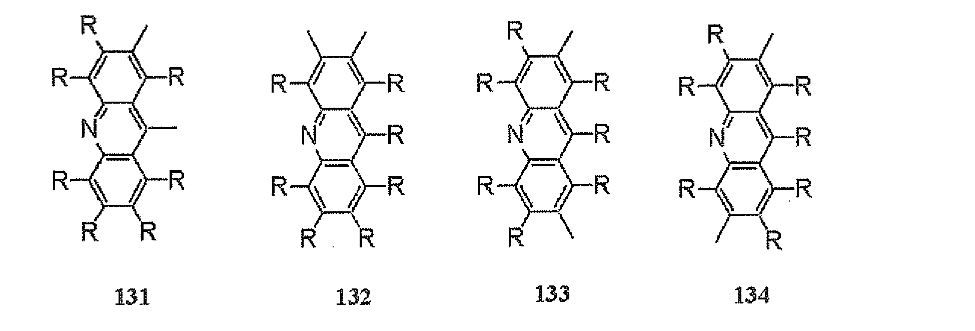

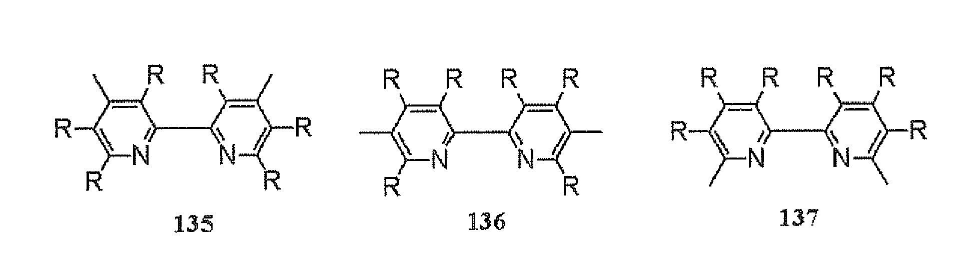

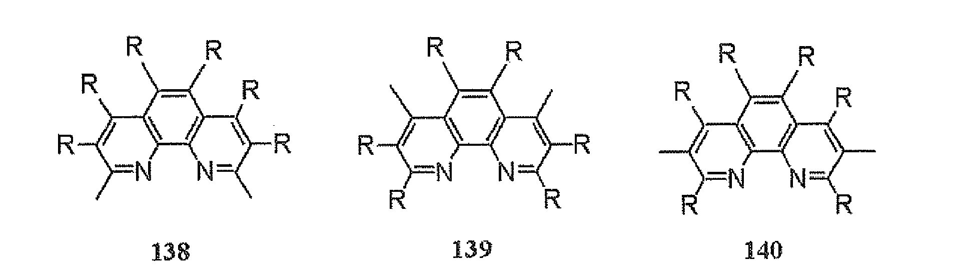

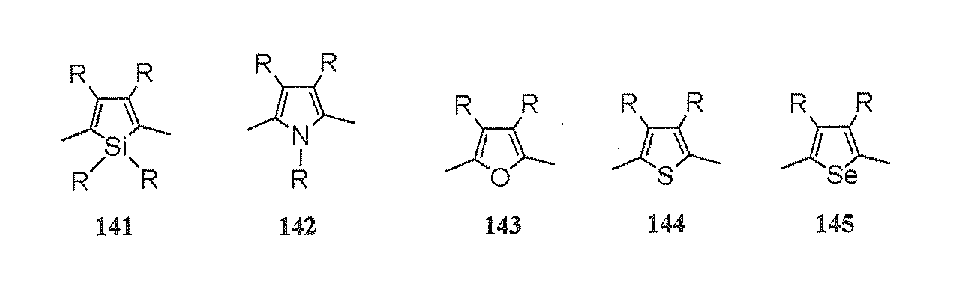

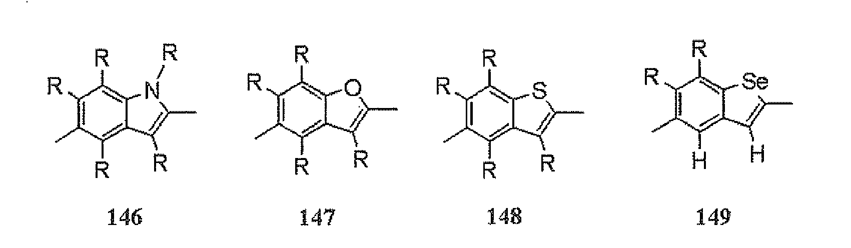

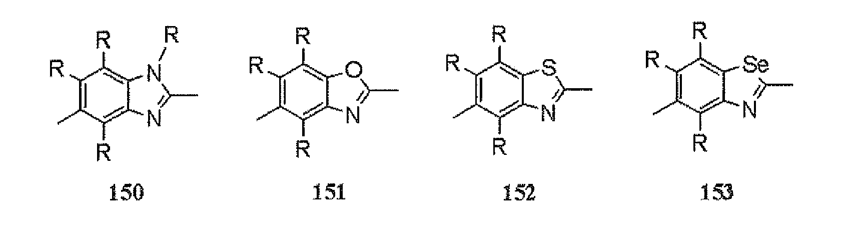

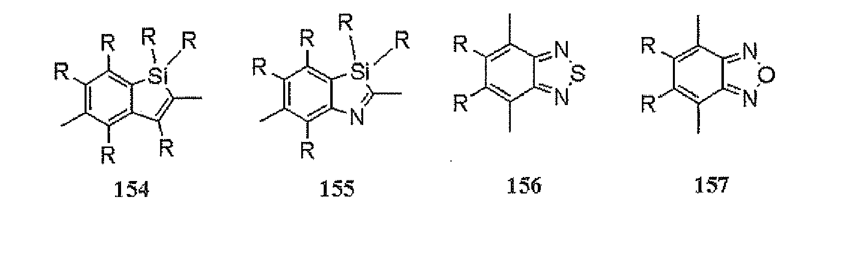

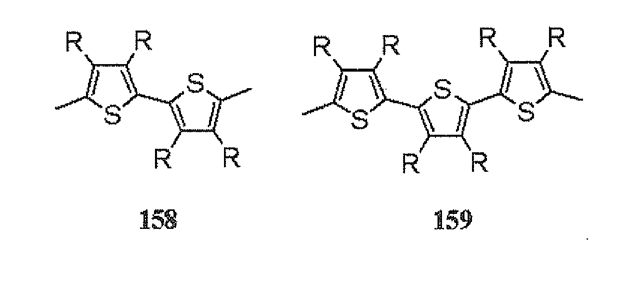

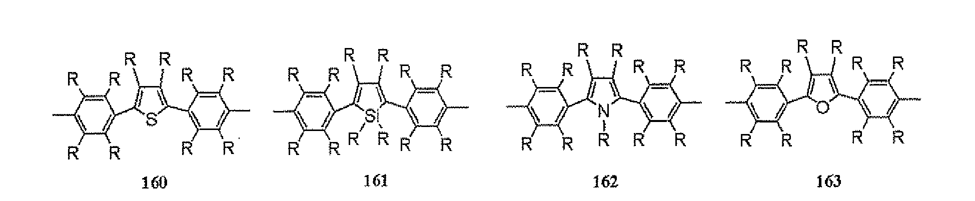

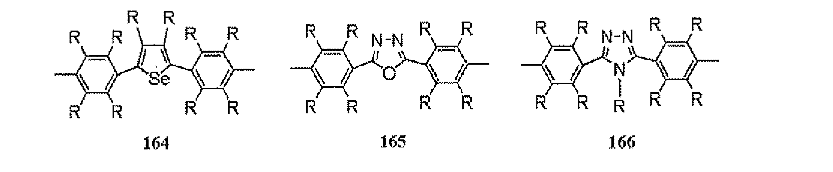

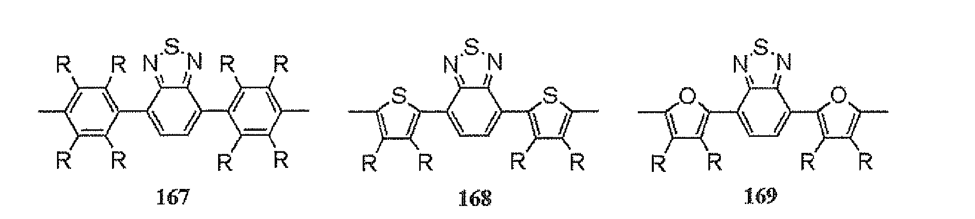

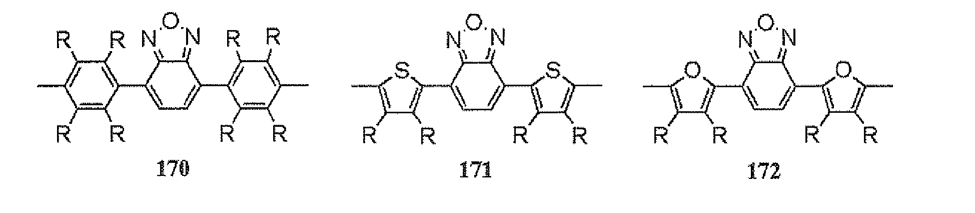

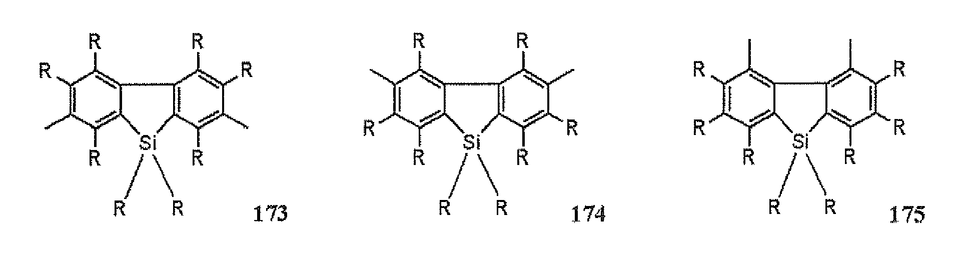

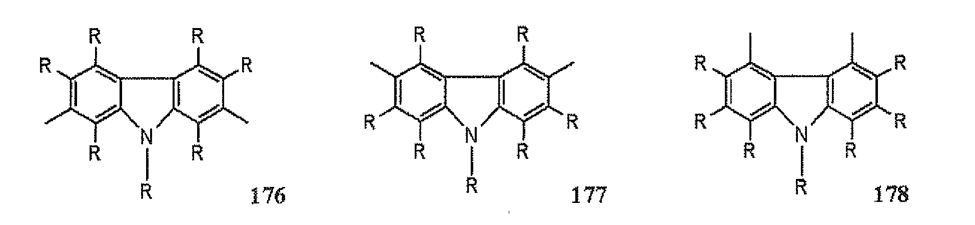

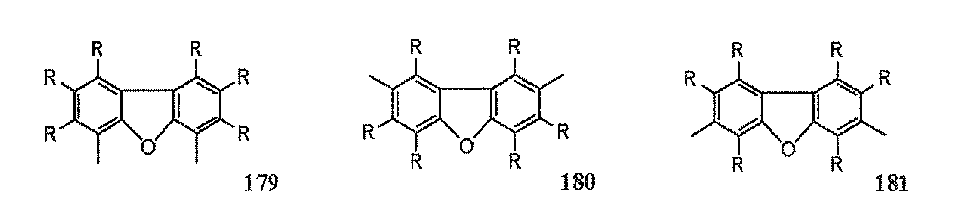

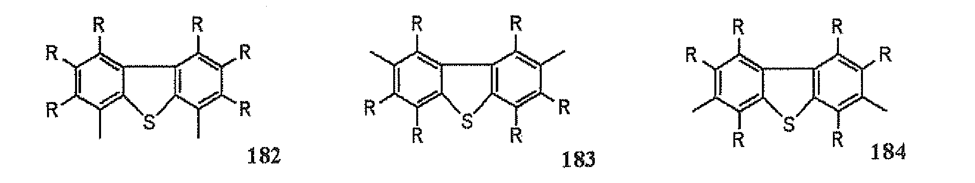

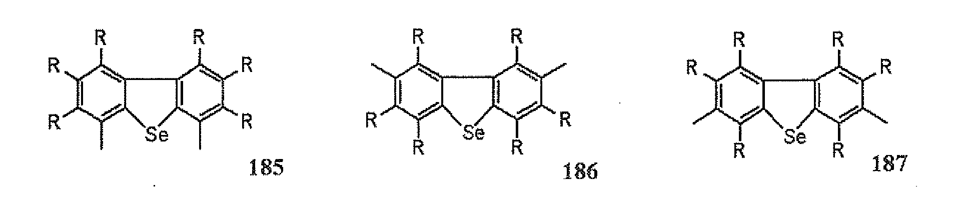

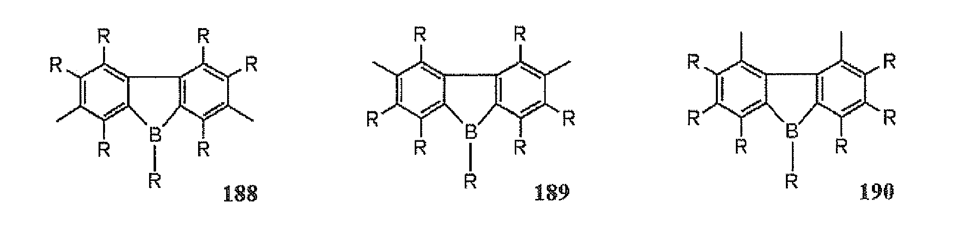

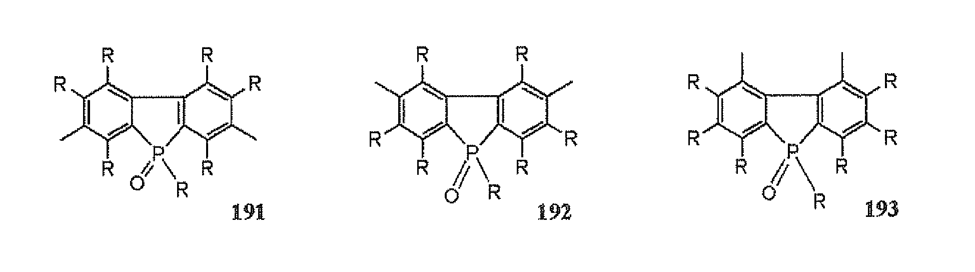

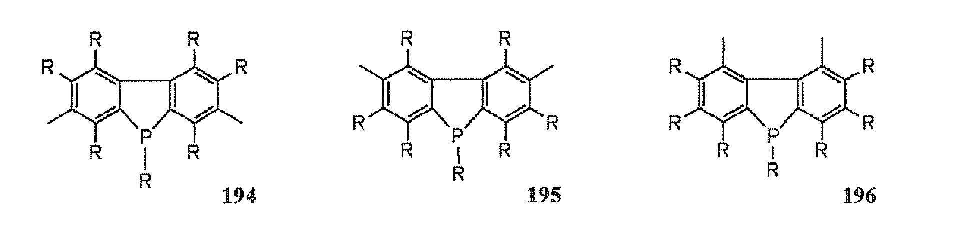

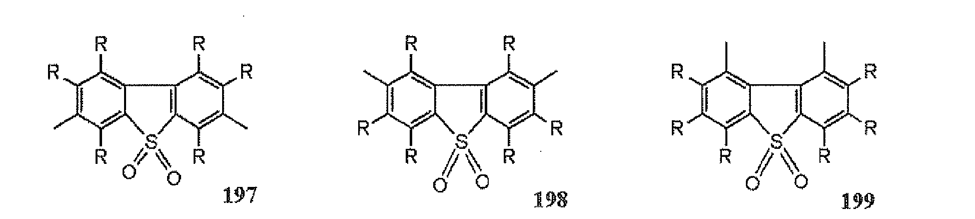

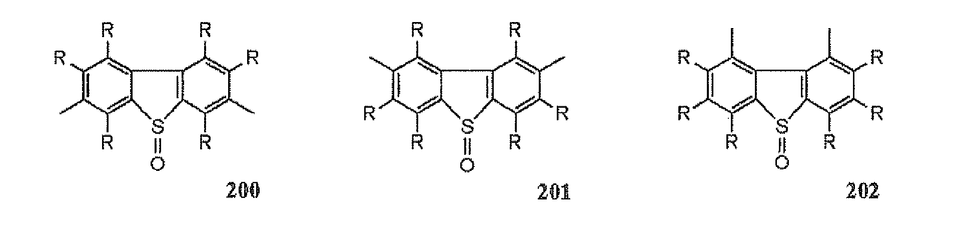

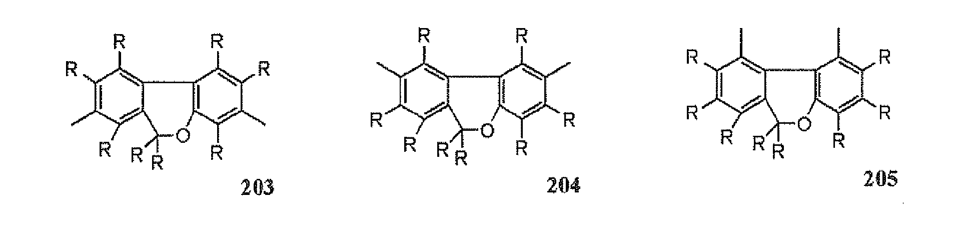

In the formulae 101 to 205, R represents a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a mono-valent heterocyclic group, a carboxyl group, a substituted carboxyl group, a cyano group or a nitro group. A plurality of Rs may be the same or different.

The definitions, examples and the like of the alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, halogen atom, acyl group, acyloxy group, imine residue, amide group, acid imide group, mono-valent heterocyclic group and substituted carboxyl group are the same as the definitions, examples and the like thereof for the above-described R¹.

From the standpoint of elongating the luminance 10% reduction lifetime when the polymer compound of the present invention is used in a polymer light emitting device, Ar¹ in the formula (I) represents preferably a group represented by the formulae 101 to 110, 126 to 130, 135 to 145, 158 to 159, 173 to 205, more preferably a group represented by the formulae 126 to 130, 135 to 137, 141 to 145, 158 to 159.

From the standpoint of easiness of synthesis of a monomer, it is preferable that the polymer compound of the present invention has a repeating unit represented by the formula (II). 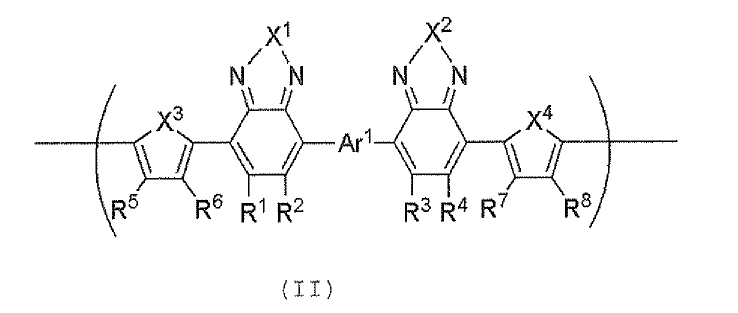 [wherein X³ and X⁴ are the same or mutually different and represent an oxygen atom, a sulfur atom or N(R^{N})-, R⁵, R⁶, R⁷ and R⁸ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and X¹, X², R¹, R², R³, R⁴, R^{N} and Ar¹ represent the same meaning as described above.]

The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group and substituted carboxyl group represented by R⁵, R⁶, R⁷ and R⁸ are the same as the definitions, examples and the like thereof for above-described R¹.

From the standpoint of easiness of synthesis of a monomer, R⁵, R⁶, R⁷ and R⁸ in the formula (II) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom or an alkyl group.

From the standpoint of easiness of synthesis of a monomer, R¹, R², R³ and R⁴ in the formula (II) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom.

From the standpoint of lowering the emission starting voltage when the polymer compound of the present invention is used in a polymer light emitting device, X¹ and X² in the (II) represent preferably a sulfur atom or N(R^{N})-, more preferably a sulfur atom.

From the standpoint of elongating the luminance 10% reduction lifetime when the polymer compound of the present invention is used in a polymer light emitting device, X³ and X⁴ in the (II) represent preferably a sulfur atom or N(R^{N})-, more preferably a sulfur atom.

From the standpoint of elongating the luminance 10% reduction lifetime when the polymer compound of the present invention is used in a polymer light emitting device, repeating units represented by the formula (III) are preferable among repeating units represented by the formula (II). 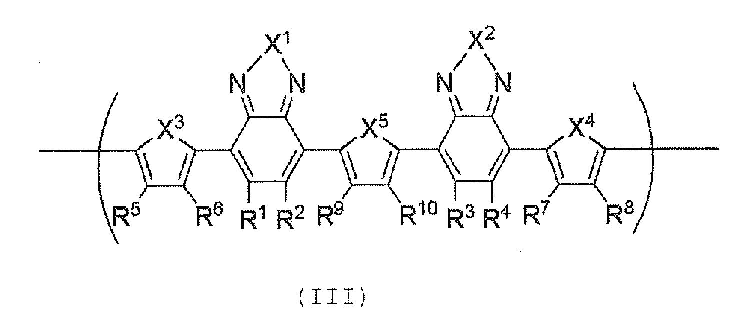 [wherein X⁵ represents an oxygen atom, a sulfur atom or N(R^{N})-, R⁹ and R¹⁰ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and X¹, X², X³, X⁴, R¹, R², R³, R⁴, R⁵, R⁶, R⁸ and R^{N} represent the same meaning as described above.].

The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group and substituted carboxyl group represented by R⁹ and R¹⁰ are the same as the definitions, examples and the like thereof for the above-described R¹.

From the standpoint of easiness of synthesis of a monomer, R⁵, R⁶, R⁷ and R⁸ in the formula (III) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom or an alkyl group.

From the standpoint of easiness of synthesis of a monomer, R¹, R², R³ and R⁴ in the formula (III) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom.

From the standpoint of easiness of synthesis of a monomer, R⁹ and R¹⁰ in the formula (III) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably an alkyl group or a hydrogen atom.

From the standpoint of lowering the emission starting voltage when the polymer compound of the present invention is used in a polymer light emitting device, X¹ and X² in the (III) represent preferably a sulfur atom or -N(R^{N})-, more preferably a sulfur atom.

From the standpoint of elongating the luminance 10% reduction lifetime when the polymer compound of the present invention is used in a polymer light emitting device, X³, X⁴ and X⁵ in the (III) represent preferably a sulfur atom or -N(R^{N})-, more preferably a sulfur atom.

Examples of the repeating unit represented by the formula (III) include repeating units represented by the formulae (III-1) to (III-22).

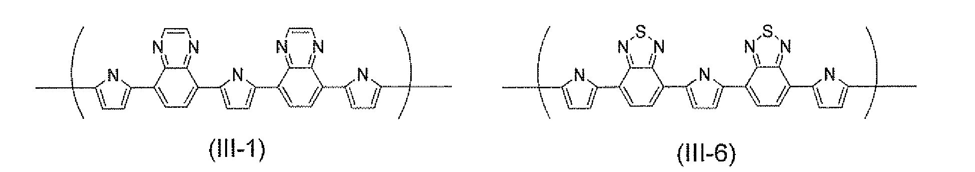

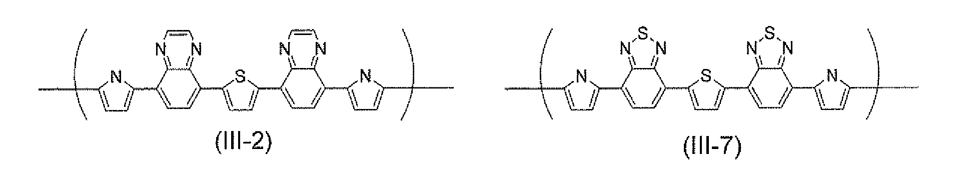

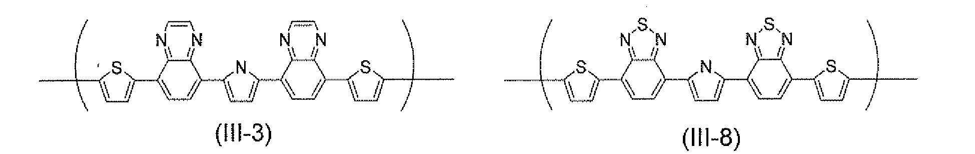

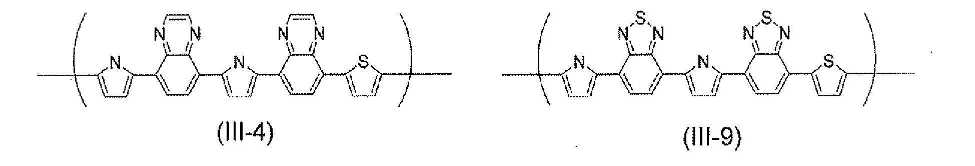

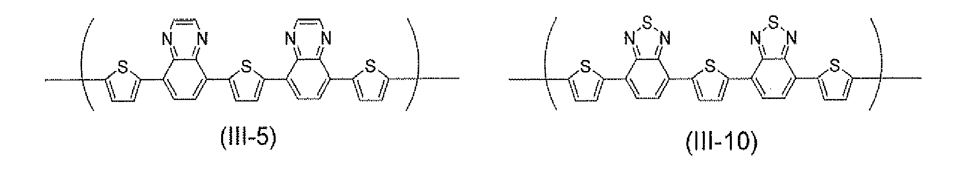

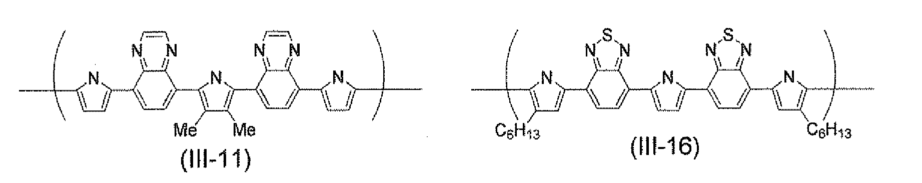

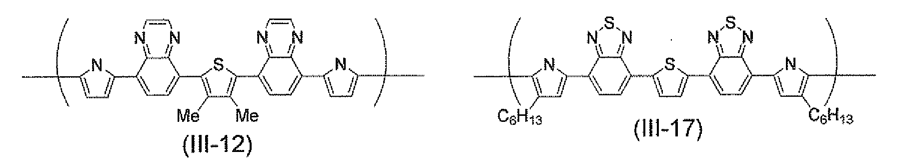

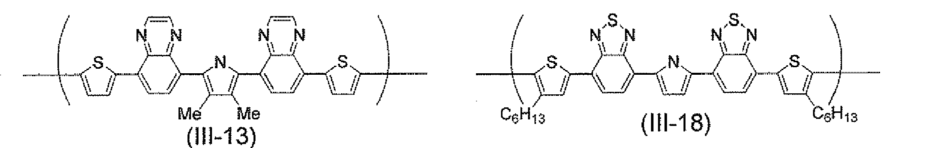

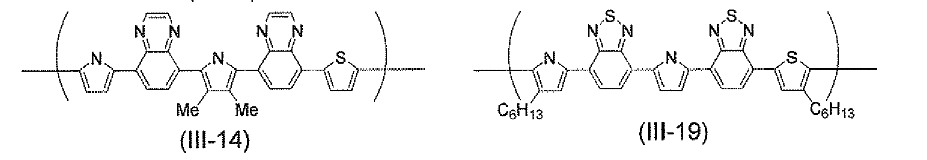

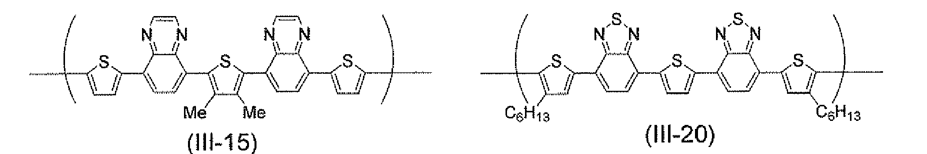

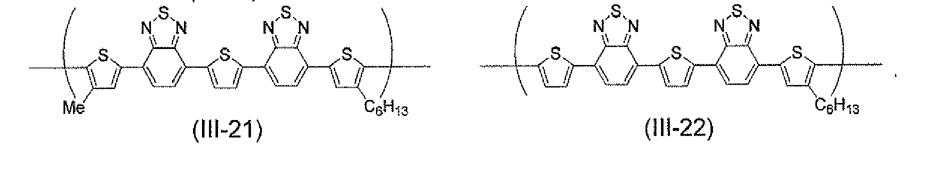

As the repeating unit represented by the formula (III), symmetric units such as repeating units represented by the formulae (III-1) to (III-13), (III-15) to (III-18) and (III-20) are preferable from the standpoint of thermal stability, and asymmetric units such as repeating units represented by the formulae (III-14), (III-19), (III-21) and (III-22) are preferable from the standpoint of solubility.

It is preferable from the standpoint of the light emission efficiency of a polymer light emitting device and device properties thereof that the polymer compound of the present invention contains further a repeating unit represented by the formula (IV). The repeating unit represented by the formula (IV) is different from the repeating unit represented by the formula (I).

-(Ar²) - (IV)

[wherein Ar² represents an arylene group, a di-valent heterocyclic group or a di-valent aromatic amine residue.].

The arylene group represented by Ar² means an atomic group obtained by removing two hydrogen atoms from an aromatic hydrocarbon, and includes also those having a condensed ring, and those having two or more independent benzene rings or condensed rings connected directly or via a vinylene group or the like. The arylene group optionally has a substituent. From the standpoint of solubility, fluorescence property, easiness of synthesis, properties when made into a device, and the like, the substituent includes preferably an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a mono-valent heterocyclic group, a carboxyl group, a substituted carboxyl group, a cyano group and a nitro group. A portion of the arylene group excluding the substituent has a carbon atom number of usually 6 to 60, preferably 6 to 20. The total carbon atom number of the arylene group including the substituent is usually 6 to 100.

Exemplified as the arylene group represented by Ar² are phenylene groups (the following formulae 1 to 3), naphthalenediyl groups (the following formulae 4 to 13), anthracene-diyl groups (the following formulae 14 to 19), biphenyl-diyl groups (the following formulae 20 to 25), terphenyl-diyl groups (the following formulae 26 to 28), fluorene-diyl groups (the following formulae 29 to 31), benzofluorene-diyl groups (the following formulae 32 to 39) and condensed ring compound groups (the following formulae 40 to 53).

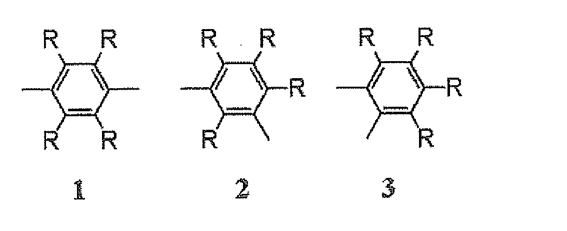

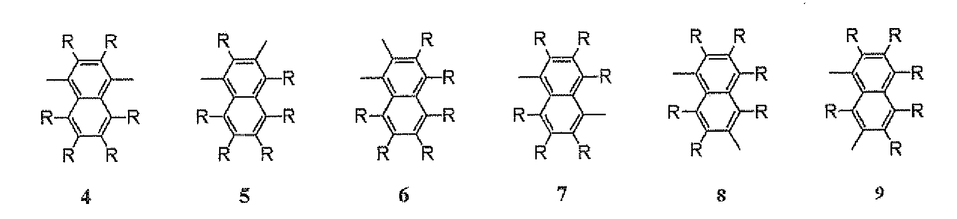

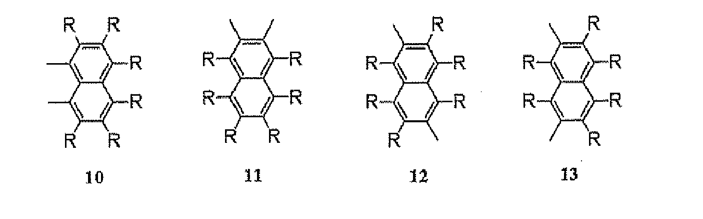

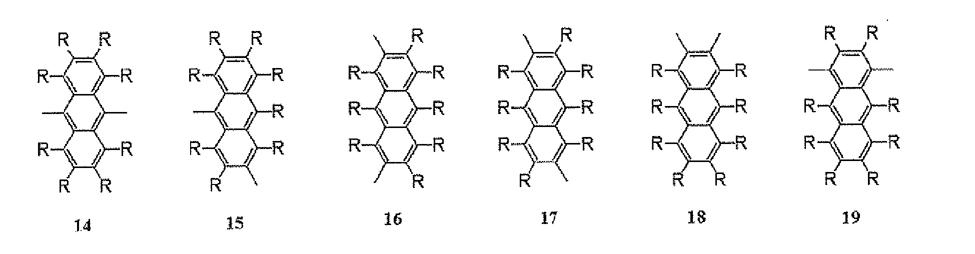

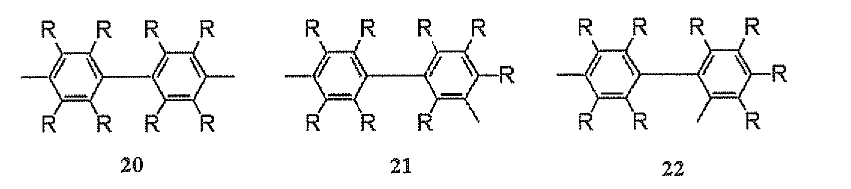

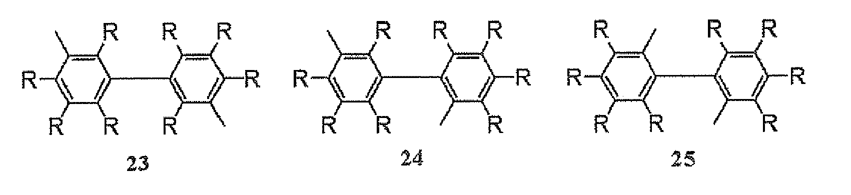

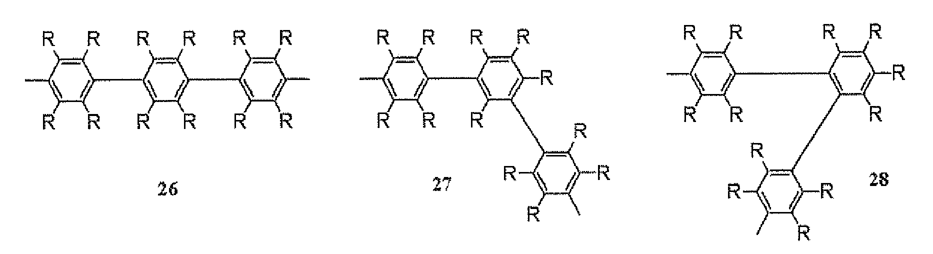

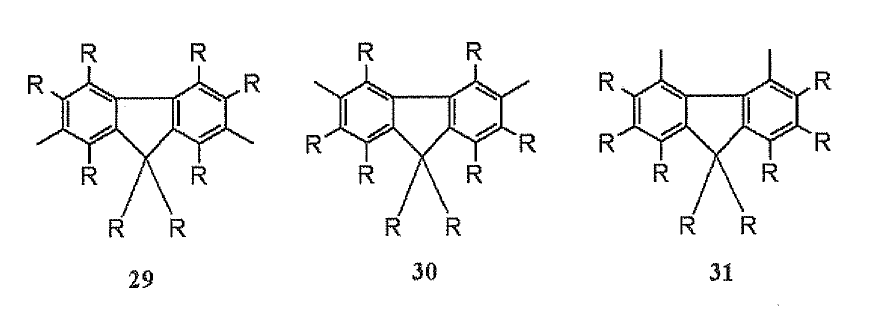

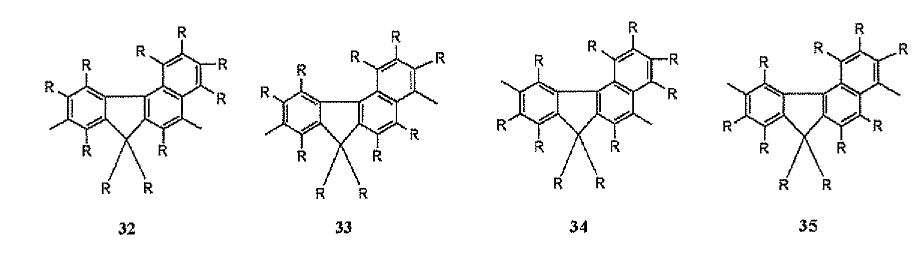

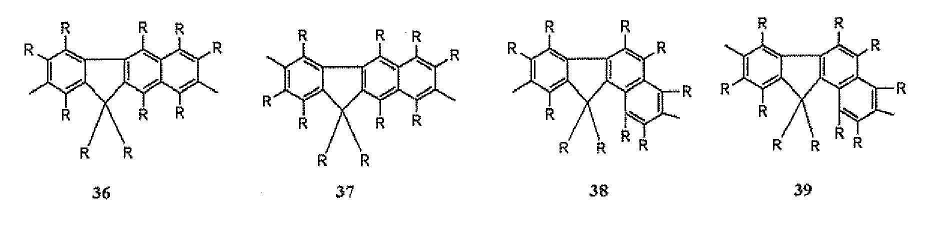

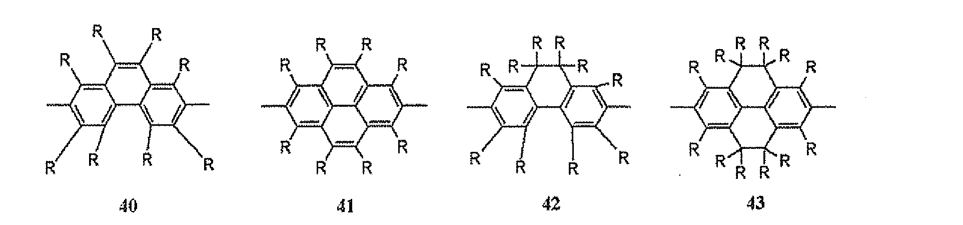

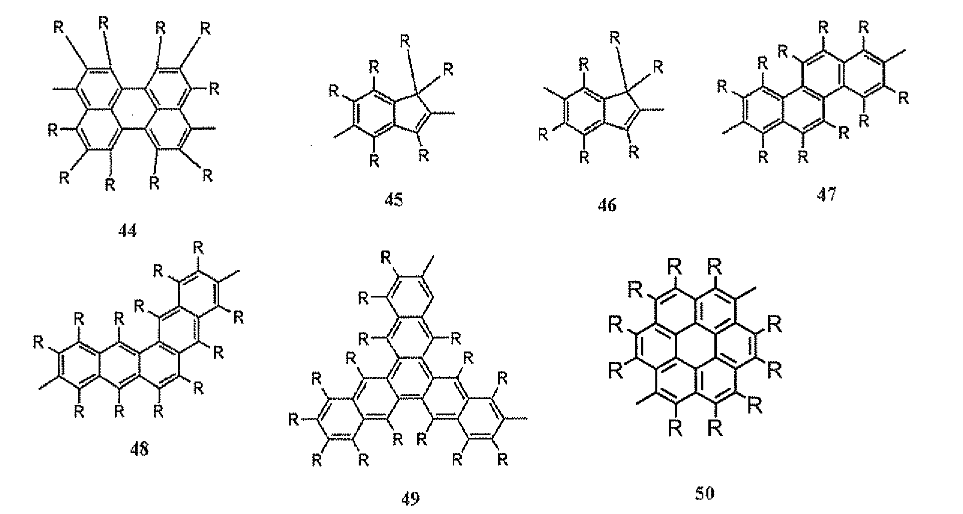

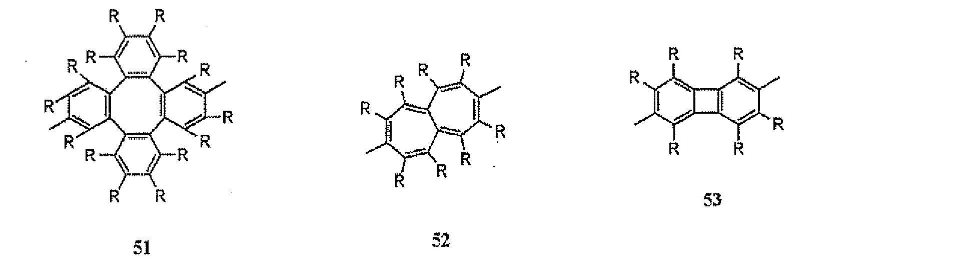

In the formulae 1 to 53, R represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a mono-valent heterocyclic group, a carboxyl group, a substituted carboxyl group, a cyano group or a nitro group. A plurality of Rs may be the same or different.

The definitions, examples and the like of the alkyl group, alkoxy group, alkylthio group, aryl group, aryloxy group, arylthio group, arylalkyl group, arylalkoxy group, arylalkylthio group, arylalkenyl group, arylalkynyl group, substituted amino group, substituted silyl group, halogen atom, acyl group, acyloxy group, imine residue, amide group, acid imide group, mono-valent heterocyclic group and substituted carboxyl group are the same as the definitions, examples and the like thereof for the above-described R¹.

The di-valent aromatic amine residue includes groups represented by the formula (VII-1), and the like. 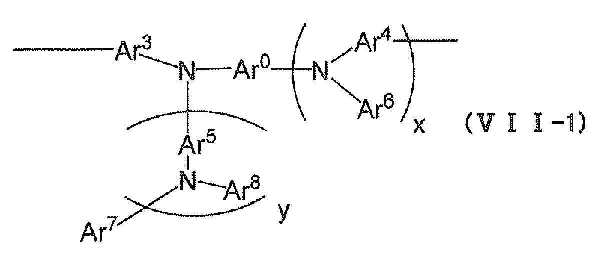 [wherein Ar⁰, Ar³, Ar⁴ and Ar⁵ are the same or mutually different and represent an arylene group or a di-valent heterocyclic group, Ar⁶, Ar⁷ and Ar⁸ are the same or mutually different and represent an aryl group or a mono-valent heterocyclic group, and x and y are the same or mutually different and represent an integer of 0 to 5. A carbon atom in Ar³ and a carbon atom in Ar⁰ may be connected directly or connected via an oxygen atom or a sulfur atom to form a condensed ring. When there exist a plurality of Ar⁴s, Ar⁵s, Ar⁶s and Ar⁸s, these may be the same or mutually different.].

The definitions, examples and the like of the arylene group and di-valent heterocyclic group are the same as the definitions, examples and the like for the above-described Ar¹. The definitions, examples and the like of the aryl group and mono-valent heterocyclic group are the same as the definitions, examples and the like for the above-described R¹.

In the (VII-1), x represents preferably an integer of 0 to 3, more preferably 0 or 1. y represents an integer of 0 to 3, more preferably 0 or 1.

Examples of the repeating unit represented by the (VII-1) include repeating units represented by the formulae (VII-1-1) to (VII-1-18).

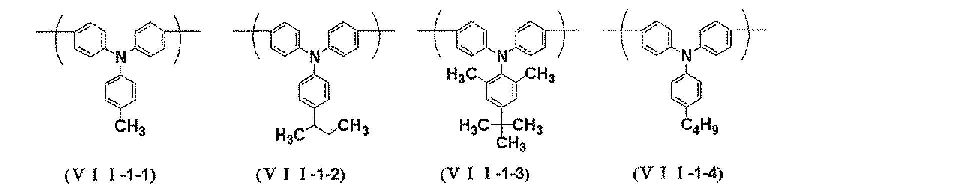

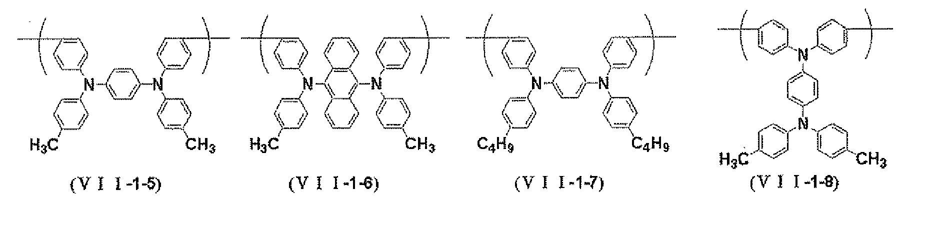

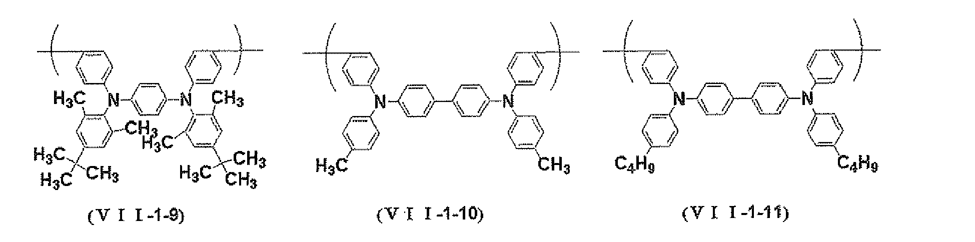

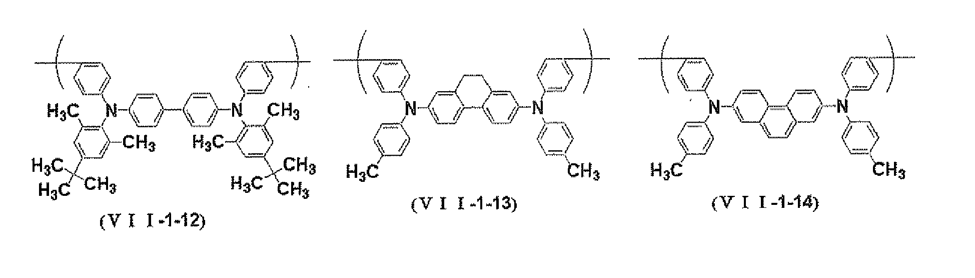

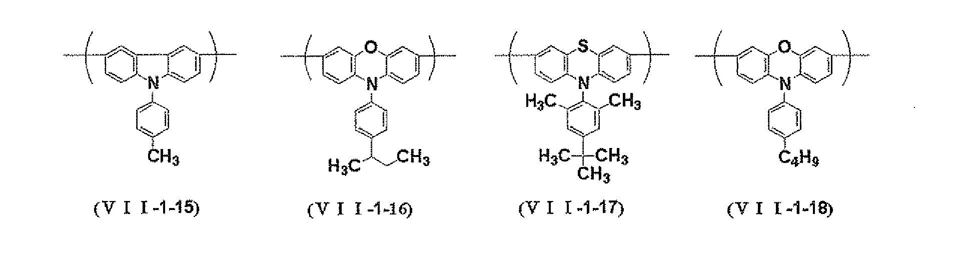

From the standpoint of the luminance 10% reduction lifetime when the polymer compound of the present invention is used in a polymer light emitting device, Ar² in the (IV) represents preferably an arylene group or a di-valent aromatic amine residue.

From the standpoint of elongating the luminance 10% reduction lifetime when the polymer compound of the present invention is used in a polymer light emitting device, the arylene group represented by Ar² in the formula (IV) is preferably a group represented by the formula (V). 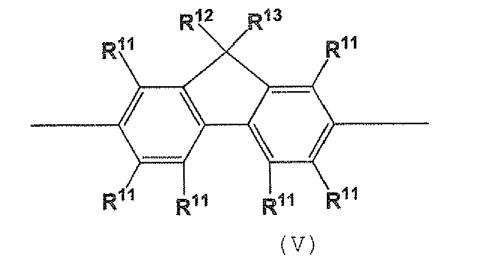 [wherein R¹² and R¹³ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an imine residue, an amide group, an acid imide group, a carboxyl group, a substituted carboxyl group, a cyano group or a nitro group. R¹¹ represents a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group. A plurality of R¹¹s may be the same or mutually different.]

The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, imine residue, amide group, acid imide group and substituted carboxyl group represented by R¹² and R¹³ are the same as the definitions, examples and the like thereof for the above-described R¹. The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group and substituted carboxyl group represented by R¹¹ are the same as the definitions, examples and the like thereof for the above-described R¹.

From the standpoint of elongating the luminance 10% reduction lifetime of a polymer light emitting device using the polymer compound of the present invention, R¹² and R¹³ in the formula (V) represent preferably an aryl group or an alkyl group.

From the standpoint of easiness of synthesis of a monomer, R¹¹ in the formula (V) represents preferably a hydrogen atom.

Examples of the group represented by the formula (V) include groups represented by the formulae (V-1) to (V-12).

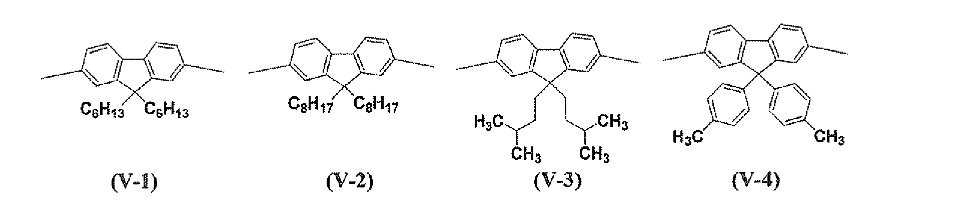

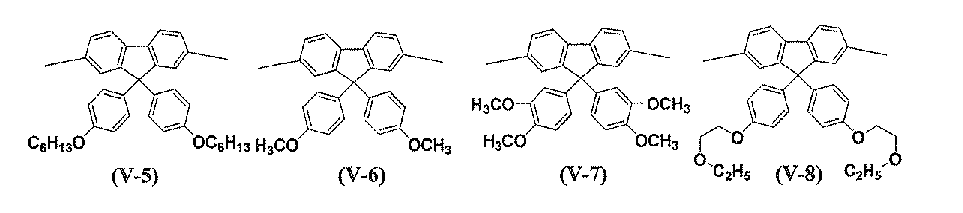

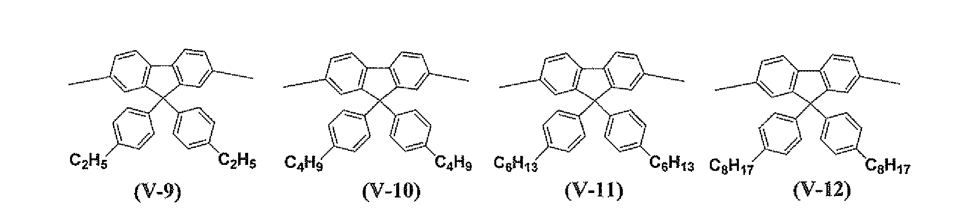

The polymer compound of the present invention preferably contains a repeating unit composed of an arylene group and a repeating unit composed of a di-valent aromatic amine residue, more preferably contains a repeating unit composed of a group represented by the above-described formula (V) and a repeating unit composed of a di-valent aromatic amine residue, as the repeating unit represented by the formula (IV), from the standpoint of elongating the luminance 10% reduction lifetime when used in a polymer light emitting device.

The polymer compound of the present invention has a polystyrene-equivalent number average molecular weight of preferably 1×10³ to 1×10⁸, more preferably 1×10³ to 1×10⁷, further preferably 1×10⁴ to 1×10⁷, from the standpoint of the lifetime property of a device. The polymer compound of the present invention has a polystyrene-equivalent weight average molecular weight of preferably 1×10³ to 1×10⁸, more preferably 1×10³ to 1×10⁷ further preferably 1×10⁴ to 1×10⁷.

The polymer compound of the present invention may be an alternative copolymer, a random copolymer, a block copolymer or a graft copolymer, or may also be a polymer having an intermediate structure thereof, for example, a random copolymer having a block property. From the standpoint of fluorescent or phosphorescent quantum yield, random copolymers having a block property and block copolymers and graft copolymers are more preferable than complete random copolymers. Further, a compound having branching in the main chain and thus having three or more end parts, and a dendrimer are also included in the polymer compound of the present invention.

As for the end group of the polymer compound of the present invention, if a polymerization active group remains intact thereon, there is a possibility of reduction in the light emitting property and lifetime when made into a device, thus, the end group may be protected with a stable group. As the end group of the polymer compound, those having a conjugated bond continuing to a conjugated structure of the main chain are preferable, and for example, there are mentioned structures connecting to an aryl group or a mono-valent heterocyclic group via a carbon-carbon bond, and substituents described as Chemical Formula 10 in JP-A No. 9-45478 are also mentioned.

As the good solvent for the polymer compound of the present invention, exemplified are chloroform, methylene chloride, dichloroethane, tetrahydrofuran, toluene, xylene, mesitylene, tetralin, decalin and n-butylbenzene. Depending on the structure and molecular weight of the polymer compound, the polymer compound can be dissolved usually in an amount of 0.1 wt% or more in these solvents.

Next, the method of producing the polymer compound of the present invention will be explained.

The polymer compound of the present invention can be produced by, for example, using a compound (monomer) represented by the formula:

Y^{a}-A-Y^{b}

[wherein -A- represents a repeating unit represented by the formula (I) or (II). Y^{a} and Y^{b} are the same or mutually different and represent a substituent participating in condensation polymerization.] as one of raw materials and condensation-polymerizing this.

The above-described substituents participating in condensation polymerization include a halogen atom, an alkyl sulfonate group, an aryl sulfonate group, an arylalkyl sulfonate group, a group derived from a borate, a sulfoniummethyl group, a phosphoniummethyl group, a phosphonate methyl group, a methyl monohalide group, -B(OH)₂, a formyl group, a cyano group, a vinyl group and the like.

The halogen atom includes a fluorine atom, a chlorine atom, a bromine atom and an iodine atom.

Exemplified as the alkyl sulfonate group are a methane sulfonate group, an ethane sulfonate group and a trifluoromethane sulfonate group.

Exemplified as the aryl sulfonate group are a benzene sulfonate group and a p-toluene sulfonate group.

Exemplified as the arylalkyl sulfonate group is a benzyl sulfonate group.

As the group derived from a borate, groups represented by the following formulae are exemplified. 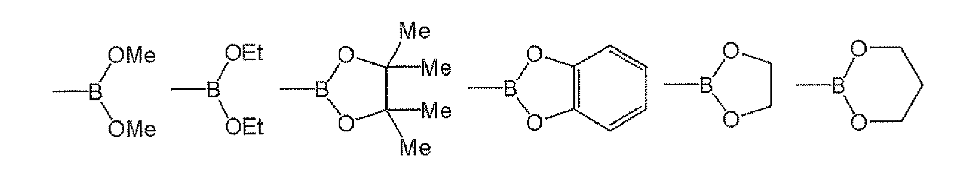 (wherein Et represents an ethyl group.)

As the sulfoniummethyl group, groups represented by the following formulae are exemplified.

-CH₂S⁺Me₂Xₐ⁻, -CH₂S⁺Ph₂Xₐ⁻

(wherein Xₐ represents a halogen atom. Ph represents a phenyl group. The same shall apply hereinafter.)

As the phosphoniummethyl group, groups represented by the following formula are exemplified.

-CH₂P⁺Ph₃Xₐ⁻

(wherein Xₐ has the same meaning as described above.)

As the phosphonate methyl group, groups represented by the following formula are exemplified.

-CH₂PO(OR')₂

(wherein R' represents an alkyl group, an aryl group or an arylalkyl group.)

Exemplified as the methyl monohalide group are a methyl fluoride group, a methyl chloride group, a methyl bromide group and a methyl iodide group.

Preferable substituents as the substituent participating in condensation polymerization vary depending on the kind of the polymerization reaction. When the polymerization reaction is, for example, a reaction using a Ni(0) complex such as the Yamamoto coupling reaction and the like, preferable are a halogen atom, an alkyl sulfonate group, an aryl sulfonate group and an arylalkyl sulfonate group. When the polymerization reaction is a reaction using a nickel catalyst or a palladium catalyst such as the Suzuki coupling reaction and the like, preferable are an alkyl sulfonate group, a halogen atom, a group derived from a borate, -B(OH)₂ and the like.

The polymer compound of the present invention can be produced, specifically, by dissolving a compound having several substituents participating in condensation polymerization as a monomer in an organic solvent if necessary, and polymerizing this at a temperature of not lower than the melting point and not higher than the boiling point of the organic solvent using, for example, an alkali and a suitable solvent. Use can be made of known methods described, for example, in Organic Reactions, Volume 14, page 270-490, John Wiley & Sons,Inc., 1965; Organic Syntheses, Collective Volume VI, page 407-411, John Wiley & Sons, Inc., 1988; Chemical Review (Chem. Rev.), Volume 95, page 2457 (1995); Journal of Organometallic Chemistry (J. Organomet. Chem.), Volume 576, page 147 (1999); Macromolecular Chemistry Macromolecular Symposium (Makromol. Chem., Macromol.Symp.), Volume 12th, page 229 (1987), and the like.

In the method of producing the polymer compound of the present invention, known condensation reactions can be used, depending on the substituent participating in condensation polymerization. For example, the correspondent monomers are subjected to: a method of polymerization by the Suzuki coupling reaction, a method of polymerization by the Grignard method, a method of polymerization with a Ni(0) complex, a method of polymerization with an oxidizer such as FeCl₃ and the like, a method of electrochemical oxidation polymerization, a method by decomposition of an intermediate polymer having a suitable leaving group, and the like. Of them, a method of polymerization by the Suzuki coupling reaction, a method of polymerization by the Grignard reaction and a method of polymerization with a nickel zero-valent complex are preferable from the standpoint of easiness of control of the structure.

Among the production methods of the polymer compound of the present invention, one of preferable embodiments is a production method in which the substituents participating in condensation polymerization are the same or mutually different and include halogen atoms, alkyl sulfonate groups, aryl sulfonate groups and arylalkyl sulfonate groups, and condensation polymerization is carried out in the present of a nickel zero-valent complex.

The above-described monomers include dihalogenated compounds, bis(alkyl sulfonate) compounds, bis(aryl sulfonate) compounds, bis(arylalkyl sulfonate) compounds, halogen-alkyl sulfonate compounds, halogen-aryl sulfonate compounds, halogen-arylalkyl sulfonate compounds, alkyl sulfonate-aryl sulfonate compounds, alkyl sulfonate-arylalkyl sulfonate compounds, and aryl sulfonate-arylalkyl sulfonate compounds. A polymer compound having a controlled sequence can be produced by using a halogen-alkyl sulfonate compound, a halogen-aryl sulfonate compound, a halogen-arylalkyl sulfonate compound, an alkyl sulfonate-aryl sulfonate compound, an alkyl sulfonate-arylalkyl sulfonate compound or an aryl sulfonate-arylalkyl sulfonate compound, as the above-described monomer.

Among the production methods of the polymer compound of the present invention, one of preferable embodiments is a production method in which the substituents participating in condensation polymerization are the same or mutually different and include halogen atoms, alkyl sulfonate groups, aryl sulfonate groups, arylalkyl sulfonate groups, -B(OH)₂ or groups derived from borates, the ratio of the sum (J) of mole numbers of halogen atoms, alkyl sulfonate groups, aryl sulfonate groups and arylalkyl sulfonate groups to the sum (K) of mole numbers of -B(OH)₂ and groups derived from borate, in all monomers, is substantially 1 (usually, K/J is in the range of 0.7 to 1.2), and condensation polymerization is carried out using a nickel catalyst or palladium catalyst.

The solvent differs depending on the reaction and the compound to be used, and for suppressing side reactions, in general, it is preferable that the solvent to be used is subjected to a sufficient deoxygenation treatment and the reaction is progressed under an inert atmosphere. Further, it is preferable to perform a dehydration treatment likewise. Here, this is not applicable to the case of a reaction in a two-phase system with water such as the Suzuki coupling reaction.

Exemplified as the solvent are saturated hydrocarbons such as pentane, hexane, heptane, octane, cyclohexane and the like, unsaturated hydrocarbons such as benzene, toluene, ethylbenzene, xylene and the like, halogenated saturated hydrocarbons such as carbon tetrachloride, chloroform, dichloromethane, chlorobutane, bromobutane, chloropentane, bromopentane, chlorohexane, bromohexane, chlorocyclohexane, bromocyclohexane and the like, halogenated unsaturated hydrocarbons such as chlorobenzene, dichlorobenzene, trichlorobenzene and the like, alcohols such as methanol, ethanol, propanol, isopropanol, butanol, t-butyl alcohol and the like, carboxylic acids such as formic acid, acetic acid, propionic acid and the like, ethers such as dimethyl ether, diethyl ether, methyl t-butyl ether, tetrahydrofuran, tetrahydropyran, dioxane and the like, amines such as trimethylamine, triethylamine, N,N,N',N'-tetramethylethylenediamine, pyridine and the like, amides such as N,N-dimethylformamide, N,N-dimethylacetamide, N,N-diethylacetamide, N-methylmorpholine oxide, and the like. These solvents may be used singly or two or more of them may be mixed and used. Of them, preferable are saturated hydrocarbons, unsaturated hydrocarabons and ethers, further preferable are hexane, octane, toluene, xylene, tetrahydrofuran and diethyl ether.

For progressing the reaction, an alkali or a suitable catalyst is added if necessary. These may be selected depending on the reaction to be used. As the alkali or catalyst, those which are sufficiently dissolved in the solvent used in the reaction are preferable. As the method of mixing an alkali or a catalyst, there is exemplified a method in which a solution of an alkali or a catalyst is added slowly to a reaction solution under an inert atmosphere such as argon, nitrogen and the like while stirring the solution, or reversely, the reaction solution is slowly added to a solution of an alkali or a catalyst.

When the polymer compound of the present invention is used in a polymer light emitting device, the purity thereof exerts an influence on a light emitting property and the like, thus, it is preferable to purify the monomer before polymerization by a method such as distillation, sublimation purification, re-crystallization and the like before performing polymerization. It is preferable, after polymerization, to carry out a purification treatment such as re-precipitation purification, chromatographic fractionation and the like.

In the production methods of the polymer compound of the present invention, a preferable embodiment is a method of producing a polymer compound having a repeating unit represented by the formula (III), comprising condensation-polymerizing a compound represented by the formula (VIII) described later in the presence of a transition metal compound. Specifically exemplified are methods in which a compound represented by the formula (VIII) described later is polymerized or a compound represented by the formula (VIII) described later and a compound having a substituent participating in condensation polymerization are polymerized, by the Suzuki coupling reaction, by the Grignard reaction, by the Yamamoto coupling reaction, by the Negishi coupling reaction, by the Stille coupling reaction, by the Ullmann coupling reaction, or with an oxidizer such as FeCl₃ and the like, and the method of polymerization by the Suzuki coupling reaction, the method of polymerization by the Grignard reaction and the method of polymerization by the Yamamoto coupling reaction are preferable from the standpoint of control of the structure.

The transition metal compound includes palladium compounds and nickel compounds in the method of polymerization by the Suzuki coupling reaction, nickel compounds in the method of polymerization by the Grignard reaction, and nickel compounds in the method of polymerization by the Yamamoto coupling reaction.

### (Compound)

Compounds useful for producing the polymer compound of the present invention include compounds represented by the formula (VI) 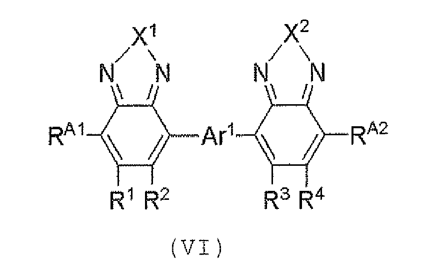 [wherein X¹and X² are the same or mutually different and represent an oxygen atom, a sulfur atom, -N(R^{N})- or C(R^{c1}=C(R^{c2})-, R¹*,* R², R³, R⁴, R^{N}, R^{A1}, R^{A2}, R^{c1} and R^{c2} are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and Ar¹represents a di-valent heterocyclic group.].

The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group and substituted carboxyl group represented by R¹, R², R³, R⁴, R^{N}, R^{c1} and R^{c2} are the same as the above-described definitions, examples and the like. The definitions, examples and the like of the di-valent heterocyclic group represented by Ar¹ are the same as the above-described definitions, examples and the like. The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group and substituted carboxyl group represented by R^{A1} and R^{A2} are the same as the definitions, examples and the like thereof for the above-described R¹.

From the standpoint of easiness of synthesis of a compound, R¹, R² , R³ and R⁴ in the formula (VI) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom.

From the standpoint of lowering the emission starting voltage when a polymer compound synthesized by using a compound represented by the formula (VI) is used in a polymer light emitting device, X¹ and X² in the formula (VI) represent preferably a sulfur atom or -N(R^{N})-, more preferably a sulfur atom.

From the standpoint of elongating the luminance 10% reduction lifetime when a polymer compound synthesized by using a compound represented by the formula (VI) is used in a polymer light emitting device, Ar¹ in the formula (VI) represents preferably a group represented by the formulae 101 to 110, 126 to 130, 135 to 145, 158 to 159 and 173 to 205, more preferably a group represented by the formulae 126 to 130, 135 to 137, 141 to 145 and 158 to 159.

R^{A1} and R^{A2} represent preferably a hydrogen atom, a halogen atom, an alkyl group, an aryl group or a mono-valent heterocyclic group, more preferably an aryl group or a mono-valent heterocyclic group.

From the standpoint of elongating the luminance 10% reduction lifetime when a polymer compound synthesized by using a compound represented by the formula (VI) is used in a polymer light emitting device, the compound represented by the formula (VI) is preferably a compound represented by the formula (VII). 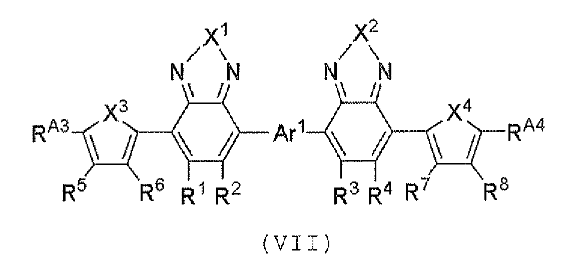 [wherein X³ and X⁴ are the same or mutually different and represent an oxygen atom, a sulfur atom or -N(R^{N})-, R^{A3}, R^{A4}, R⁵, R⁶ , R⁷ and R⁸ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and X¹, X², R¹, R², R³ , R⁴, R^{N} and Ar¹ represent the same meaning as described above.].

The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group and substituted carboxyl group represented by R⁵, R⁶ , R⁷ and R⁸ are the same as the above-described definitions, examples and the like. The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group and substituted carboxyl group represented by R^{A3} and R^{A4} are the same as the definitions, examples and the like thereof for the above-described R¹.

From the standpoint of easiness of synthesis of a compound, R⁵ , R⁶ , R⁷ and R⁶ in the formula (VII) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom or an alkyl group.

From the standpoint of easiness of synthesis of a compound, R¹, R², R³ and R⁴ in the (VII) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom.

From the standpoint of lowering the emission starting voltage when a polymer compound synthesized by using a compound represented by the formula (VII) is used in a polymer light emitting device, X¹ and X² in the formula (VII) represent preferably a sulfur atom or -N(R^{N})-, more preferably a sulfur atom.

From the standpoint of elongating the luminance 10% reduction lifetime when a polymer compound synthesized by using a compound represented by the formula (VII) is used in a polymer light emitting device, X³ and X⁴ in the formula (VII) represent preferably a sulfur atom or -N(R^{N})-, more preferably a sulfur atom.

R^{A3} and R^{A4} represent preferably a hydrogen atom, a halogen atom, an alkyl group or an aryl group, more preferably a hydrogen atom or a halogen atom.

From the standpoint of elongating the luminance 10% reduction lifetime when a polymer compound synthesized by using a compound represented by the formula (VII) is used in a polymer light emitting device, the compound represented by the formula (VII) is preferably a compound represented by the formula (VIII). 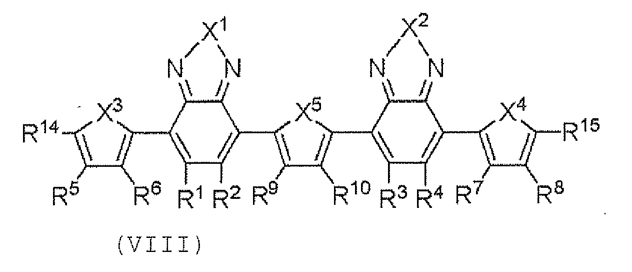 [wherein X⁵ represents an oxygen atom, a sulfur atom or -N(R^{N})-, R⁹, R¹⁰, R¹⁴ and R¹⁵ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group, a nitro group or a group derived from a borate, and X¹, X², X³, X⁴, R¹, R², R³, R⁴, R⁵, R⁶ , R⁷, R⁸ and R^{N} represent the same meaning as described above.].

The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group and substituted carboxyl group represented by R⁹ and R¹⁰ are the same as the above-described definitions, examples and the like. The definitions, examples and the like of the halogen atom, alkyl group, alkoxy group, alkylthio group, aryl group, aryloxy group, arylthio group, arylalkyl group, arylalkoxy group, arylalkylthio group, arylalkenyl group, arylalkynyl group, mono-valent heterocyclic group, heterocyclic thio group, substituted amino group, substituted silyl group, acyl group, acyloxy group, imine residue, amide group, acid imide group, substituted carboxyl group and group derived from a borate represented by R¹⁴ and R¹⁵ are the same as the definitions, examples and the like thereof for the above-described R¹.

From the standpoint of easiness of synthesis of a compound, R⁵, R⁶, R⁷ and R⁸ in the (VIII) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom or an alkyl group.

From the standpoint of easiness of synthesis of a compound, R¹, R², R³ and R⁴ in the (VIII) represent preferably a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group or a mono-valent heterocyclic group, more preferably a hydrogen atom, an alkyl group or an aryl group, further preferably a hydrogen atom.

From the standpoint of easiness of synthesis of a compound, R⁹ and R¹⁰ in the formula (VIII) represent preferably a hydrogen atom, an alkyl group, an alkoxy group, an alkylthio group, an aryl group, an aryloxy group, an arylthio group, an arylalkyl group, n arylalkoxy group, an arylalkylthio group, an arylalkenyl group, an arylalkynyl group or a mono-valent heterocyclic group, more preferably a hydrogen atom, an alkyl group or an aryl group, further preferably an alkyl group or a hydrogen atom.

From the standpoint of lowering the emission starting voltage when a polymer compound synthesized by using a compound represented by the formula (VIII) is used in a polymer light emitting device, X¹ and X² in the (VIII) represent preferably a sulfur atom or - N(R^{N})-, more preferably a sulfur atom.

From the standpoint of elongating the luminance 10% reduction lifetime when a polymer compound synthesized by using a compound represented by the formula (VIII) is used in a polymer light emitting device, X³, X⁴ and X⁵ in the formula (VIII) represent preferably a sulfur atom or -N(R^{N})-, more preferably a sulfur atom.

R¹⁴ and R¹⁵ represent preferably a hydrogen atom, a halogen atom, an alkyl group or an aryl group, more preferably a hydrogen atom or a halogen atom.

Examples of the compound represented by the formula (VIII) include compounds represented by the formulae (VIII-1) to (VIII-8).

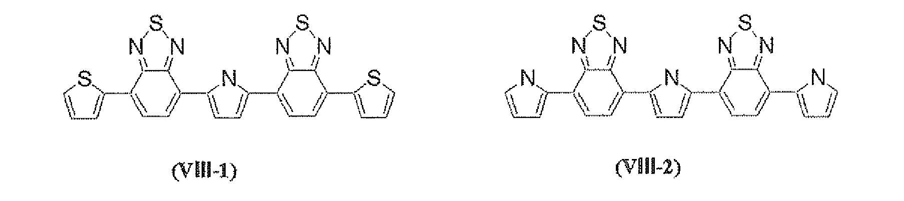

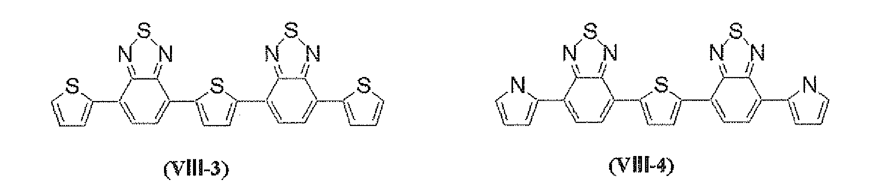

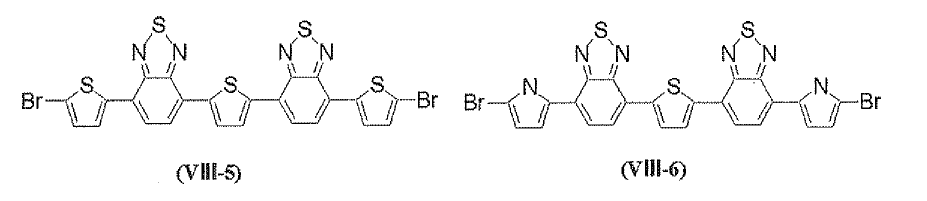

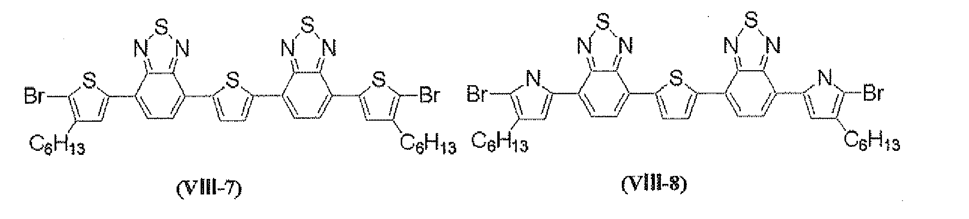

The compound represented by the formula (VIII) can be synthesized, for example, by the following method. 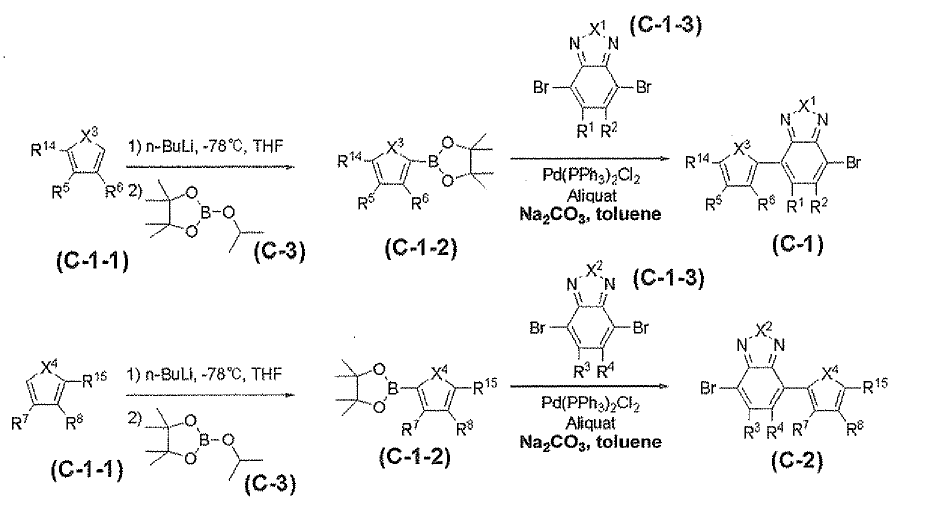 (wherein n-Bu represents a n-butyl group, the same shall apply hereinafter.)

A compound (C-1-1) can be reacted with a compound (C-3) in the presence of a n-butyllithium hexane solution in a tetrahydrofuran solvent, to synthesize a borate body (C-1-2). Further, the Suzuki coupling reaction can be carried out with a dibromo body (C-1-3) in a toluene solvent in the presence of a palladium catalyst and a base, to synthesize a compound (C-1). According to the same procedure, a compound (C-2) can be synthesized from the compound (C-1-1) via the above-described route. 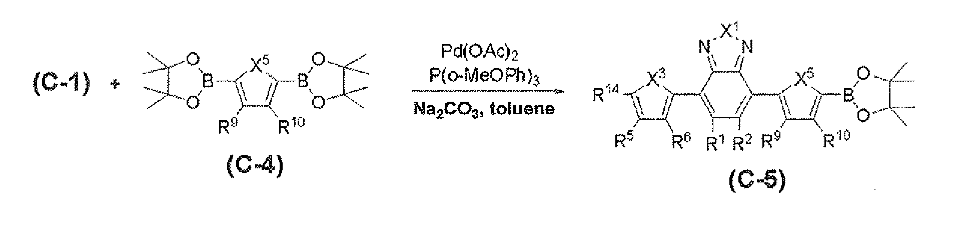 (wherein Ac represents an acetyl group, the same shall apply hereinafter.)

The compound (C-1) obtained above and a diborate body (C-4) can be subjected to the Suzuki coupling reaction in a toluene solvent in the presence of a palladium catalyst and a base, to synthesize a compound (C-5).

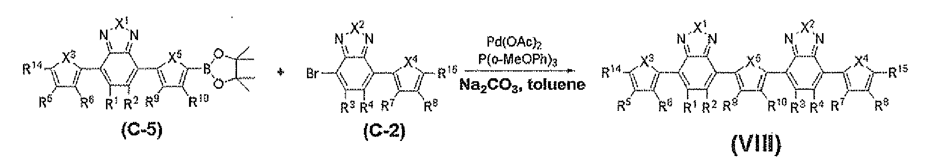

The compound (C-5) and the compound (C-2) obtained above can be subjected to the Suzuki coupling reaction in a toluene solvent in the presence of a palladium catalyst and a base, to obtain a compound represented by the formula (VIII).

From the standpoint of elongating the luminance 10% reduction lifetime when a polymer compound synthesized by using the compound represented by the formula (VIII) is used in a polymer light emitting device, the compound represented by the formula (VIII) is preferably a compound represented by the formula (IX). 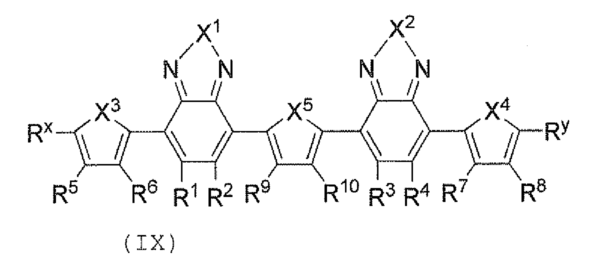 [wherein R^{x} and R^{y} are the same or mutually different and represent a halogen atom, and X¹, X², X³, X⁴, X⁵, R¹, R², R³, R⁴, R⁵, R⁶, R⁷, R⁸, R⁹, R¹⁰ and R^{N} represent the same meaning as described above.].

The halogen atom represented by R^{x} and R^{y} in the formula (IX) includes a fluorine atom, a chlorine atom, a bromine atom and an iodine atom.

### (Composition)

The composition of the present invention is a composition containing the polymer compound of the present invention, and includes a composition containing at least one material selected from the group consisting of a hole transporting material, an electron transporting material and a light emitting material, and containing at least one polymer compound of the present invention, a composition containing at least two polymer compounds of the present invention, and the like.

The composition of the present invention may be a liquid composition, and is useful for fabrication of light emitting devices such as polymer light emitting devices, and organic transistors. The liquid composition contains the above-described polymer compound and a solvent. In this specification, "liquid composition" means a composition which is liquid in device fabrication, and usually, is liquid at normal pressure (namely, 1 atm) and 25°C. The liquid composition is, in general, referred to as ink, ink composition, solution or the like in some cases.

The liquid composition may also contain a low molecular weight light emitting material, a hole transporting material, an electron transporting material, a stabilizer, additives for adjusting viscosity and/or surface tension, an antioxidant and the like, in addition to the above-described polymer compound. These optional components may be used each singly or in combination of two or more.

Examples of the low molecular weight fluorescent material which may be contained in the liquid composition include low molecular weight fluorescent materials such as naphthalene derivatives, anthracene, anthracene derivatives, perylene, perylene derivatives, polymethine dyes, xanthene dyes, coumarine dyes, cyanine dyes; metal complexes having a metal complex of 8-hydroxyquinoline as a ligand; metal complexes having a 8-hydroxyquinoline derivative as a ligand; other fluorescent metal complexes, aromatic amines, tetraphenylcyclopentadiene, tetraphenylcyclopentadiene derivatives, tetraphenylcyclobutadiene, tetraphenylcyclobutadiene derivatives, stilbenes, silicon-containing aromatics, oxazoles, furoxanes, thiazoles, tetraarylmethanes, thiadiazoles, pyrazoles, metacyclophanes, acetylenes and the like. Specifically, materials described in, for example, JP-A No. 57-51781, JP-A No. 59-194393 and the like, and known materials are mentioned.

Examples of the hole transporting material which may be contained in the liquid composition include polyvinylcarbazole and derivatives thereof, polysilane and derivatives thereof, polysiloxane derivatives having an aromatic amine on the side chain or the main chain, pyrazoline derivatives, arylamine derivatives, stilbene derivatives, triphenyldiamine derivatives, polyaniline and derivatives thereof, polythiophene and derivatives thereof, polypyrrole and derivatives thereof, poly(p-phenylenevinylene) and derivatives thereof, and poly(2,5-thienylenevinylene) and derivatives thereof.

Examples of the electron transporting material which may be contained in the liquid composition include oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinoline derivatives; metal complexes of 8-hydroxyquinoline and derivatives thereof; polyquinoline and derivatives thereof, polyquinoxaline and derivatives thereof, and polyfluorene and derivatives thereof.

Examples of the stabilizer which may be contained in the liquid composition include phenol antioxidants and phosphorus antioxidants.

Examples of the additive for adjusting viscosity and/or surface tension which may be contained in the liquid composition include a high molecular weight compound (thickening agent) and a poor solvent for increasing viscosity, a low molecular weight compound for decreasing viscosity, and a surfactant for lowering surface tension. The poor solvent means a solvent with which the weight of the polymer compound of the present invention dissolvable in 1 g of the solvent is 0.1 mg or less.

As the above-described high molecular weight compound, those not disturbing light emission and charge transportation may be permissible, and usually, those which are soluble in the solvent of the liquid composition are mentioned. As the high molecular weight compound, for example, polystyrene of high molecular weight and polymethyl methacrylate of high molecular weight can be used. The above-described high molecular weight compound has a polystyrene-equivalent weight average molecular weight of preferably 500000 or more, more preferably 1000000 or more. A poor solvent can also be used as a thickening agent.

As the antioxidant which may be contained in the liquid composition, those not disturbing light emission and charge transportation may be permissible, and when the composition contains a solvent, usually, those which are soluble in the solvent are mentioned. As the antioxidant, phenol antioxidants and phosphorus antioxidants are exemplified. By use of the antioxidant, the preservation stability of the above-described polymer compound and solvent can be improved.

When the liquid composition contains a hole transporting material, the amount of the hole transporting material in the liquid composition is usually 1 to 80 parts by weight, preferably 5 to 60 parts by weight when the weight of all components excluding the solvent is 100 parts by weight. When the liquid composition of the present invention contains an electron transporting material, the amount of the electron transporting material in the liquid composition is usually 1 to 80 parts by weight, preferably 5 to 60 parts by weight when the weight of all components excluding the solvent is 100 parts by weight.

In the case of firm formation using this liquid composition in fabricating a polymer light emitting device, it may be advantageous to only remove a solvent by drying after application of the liquid composition, and also in the case of mixing of a charge transporting material and a light emitting material, the same means can be applied, that is, this method is extremely advantageous for production. In drying, drying may be effected under heating at about 50 to 150°C, alternatively, drying may be carried out under reduced pressure of about 10⁻³ Pa.

As the film formation method using the liquid composition, application methods such as a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet print method and the like can be used.

The proportion of a solvent in the liquid composition is usually 1 wt% to 99.9 wt%, preferably 60 wt% to 99.9 wt%, further preferably 90 wt% to 99.8 wt% with respect to the total weight of the liquid composition. Though the viscosity of the liquid composition varies depending on a printing method, the viscosity at 25°C is preferably in the range of 0.5 to 500 mPa·s, and when a liquid composition passes through a discharge apparatus such as in an inkjet print method and the like, the viscosity at 25°C is preferably in the range of 0.5 to 20 mPa·s, for preventing clogging and flying curving in discharging.

As the solvent contained in the liquid composition, those capable of dissolving or dispersing components other than the solvent in the composition are preferable. Exemplified as the solvent are chlorine-based solvents such as chloroform, methylene chloride, 1,2-dichloroethane, 1,1,2-trichloroethane, chlorobenzene, o-dichlorobenzene and the like, ether solvents such as tetrahydrofuran, dioxane and the like, aromatic hydrocarbon solvents such as toluene, xylene, trimethylbenzene, mesitylene and the like, aliphatic hydrocarbon solvents such as cyclohexane, methylcyclohexane, n-pentane, n-hexane, n-heptane, n-octane, n-nonane, n-decane and the like, ketone solvents such as acetone, methyl ethyl ketone, cyclohexanone and the like, ester solvents such as ethyl acetate, butyl acetate, methyl benzoate, ethylcellosolve acetate and the like, polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexane diol and the like and derivatives thereof, alcohol solvents such as methanol, ethanol, propanol, isopropanol, cyclohexanol and the like, sulfoxide solvents such as dimethyl sulfoxide and the like, amide solvents such as N-methyl-2-pyrrolidone, N,N-dimethylformamide, and the like. These solvents may be used singly or in combination of two or more. Among the above-described solvents, one or more organic solvents having a structure containing at least one benzene ring and having a melting point of 0°C or lower and a boiling point of 100°C or higher are preferably contained from the standpoint of viscosity, film formability and the like.

Regarding the kind of the solvent, aromatic hydrocarbon solvents, aliphatic hydrocarbon solvents, ester solvents and ketone solvents are preferable from the standpoint of solubility of components other than the solvent in the liquid composition into the solvent, uniformity in film formation, viscosity property and the like, and more preferable are toluene, xylene, ethylbenzene, diethylbenzene, trimethylbenzene, mesitylene, n-propylbenzene, i-propylbenzene, n-butylbenzene, i-butylbenzene, s-butylbenzene, anisole, ethoxybenzene, 1-methylnaphthalene, cyclohexane, cyclohexanone, cyclohexylbenzene, bicyclohexyl, cyclohexenylcyclohexanone, n-heptylcyclohexane, n-hexylcyclohexane, methyl benzoate, 2-propylcyclohexanone, 2-heptanone, 3-heptanone, 4-heptanone, 2-octanone, 2-nonanone, 2-decanone, dicyclohexyl ketone and bicyclohexylmethyl benzoate, and particularly preferable are xylene, anisole, mesitylene, cyclohexylbenzene and bicyclohexylmethyl benzoate.

The number of the solvent to be contained in the liquid composition is preferably 2 or more, more preferably 2 to 3, and further preferably 2 from the standpoint of film formability and from the standpoint of device properties, and the like.

When two solvents are contained in the liquid composition, one of them may be solid at 25°C. From the standpoint of film formability, it is preferable that one solvent has a boiling point of 180°C or higher and another solvent has a boiling point of lower than 180°C, and it is more preferable that one solvent has a boiling point of 200°C or higher and another solvent has a boiling point of lower than 180°C.

When three solvents are contained in the liquid composition, one or two of them may be solid at 25°C. From the standpoint of film formability, it is preferable that at least one of three solvents has a boiling point of 180°C or higher and at least one solvent has a boiling point of 180°C or lower, and it is more preferable that at least one of three solvents has a boiling point of 200°C or higher and 300°C or lower and at least one solvent has a boiling point of 180°C or lower.

When two or more solvents are contained in the liquid composition, the content of a solvent having the highest boiling point is preferably 40 to 90 wt%, more preferably 50 to 90 wt%, and further preferably 65 to 85 wt% with respect to the weight of all solvents contained in the liquid composition, from the standpoint of viscosity and film formability.

### <Application>

The polymer compound of the present invention can be used not only as a light emitting material, but also as a film material, an organic semiconductor material, an organic transistor material, an optical material, a solar battery material, or a conductive material by doping.

The film of the present invention will be illustrated. This film is obtained by using the above-described polymer compound. As the kind of the film, a light emitting film, a conductive film, an organic semiconductor film and the like are exemplified.

The light emitting film has a light emission quantum yield of preferably 50% or more, more preferably 60% or more and further preferably 70% or more from the standpoint of the luminance and light emission voltage of a device, and the like.

The conductive film preferably has a surface resistance of 1 KΩ/□ or less. By doping the film with a Lewis acid, an ionic compound or the like, electric conductivity can be enhanced. The surface resistance is more preferably 100 KΩ/□ or less, further preferably 10 KΩ/□ or less.

In the organic semiconductor film, one larger parameter of electron mobility or hole mobility is preferably 10⁻⁵ cm²/V/s or more, more preferably 10⁻³ cm²/V/s or more, and further preferably 10⁻¹ cm²/V/s or more. Using the organic semiconductor film, an organic transistor can be fabricated. Specifically, by forming the organic semiconductor film on a Si substrate carrying a gate electrode and an insulation film made of SiO₂ and the like formed thereon, and forming a source electrode and a drain electrode with Au and the like, an organic transistor can be obtained.

Next, the polymer light emitting device of the present invention will be described.

The polymer light emitting device of the present invention has an anode, a cathode, and an organic layer containing the above-described polymer compound and disposed between the anode and the cathode. The above-described organic layer functions as a light emitting layer, a hole transporting layer, a hole injection layer, an electron transporting layer, an electron injection layer or the like. In the polymer light emitting device of the present invention, the above-described organic layer is preferably a light emitting layer.

The polymer light emitting device of the present invention includes (1) a polymer light emitting device having an electron transporting layer disposed between a cathode and a light emitting layer; (2) a polymer light emitting device having a hole transporting layer disposed between an anode and a light emitting layer; (3) a polymer light emitting device having an electron transporting layer disposed between a cathode and a light emitting layer and having a hole transporting layer disposed between an anode and a light emitting layer; and the like.

More specifically, the following structures a) to d) are exemplified.
a) anode/light emitting layer/cathode
b) anode/hole transporting layer/light emitting layer/cathode
c) anode/light emitting layer/electron transporting layer/cathode
d) anode/hole transporting layer/light emitting layer/electron transporting layer/cathode
(wherein, "/" means adjacent lamination of layers; the same shall apply hereinafter.)

The light emitting layer is a layer having a function of emitting light. The hole transporting layer is a layer having a function of transporting holes, and the electron transporting layer is a layer having a function of transporting electrons. The electron transporting layer and the hole transporting layer are collectively called a charge transporting layer. The light emitting layer, hole transporting layer and electron transporting layer are the same or mutually different, and two or more layers of each of them may be used.

A hole transporting layer adjacent to a light emitting layer is called an interlayer layer in some cases.

As the method of film formation of a light emitting layer, methods of film formation from a solution are exemplified.

For film formation from a solution, application methods such as a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet print method and the like can be used.

In the case of film formation from a solution using the polymer compound of the present invention in fabricating a polymer light emitting device, it may be advantageous to only remove a solvent by drying after application of this solution, and also in the case of mixing of a charge transporting material and a light emitting material, the same means can be applied, that is, this method is extremely advantageous for production.

The film thickness of a light emitting layer shows an optimum value varying depending on the material to be used, and may be advantageously selected so as to give appropriate values of driving voltage and light emission efficiency, and is usually 1 nm to 1 µm, preferably 2 nm to 500 nm, further preferably 5 nm to 200 nm.

In the polymer light emitting device of the present invention, a light emitting material other than the above-described polymer compound may be mixed and used in a light emitting layer. In the polymer light emitting device of the present invention, the light emitting layer containing a light emitting material other than the above-described polymer compound may be laminated with a light emitting layer containing the above-described polymer compound.

As the light emitting material other than the above-described polymer compound, known compounds can be used. Examples of the low molecular weight compound include naphthalene derivatives, anthracene and derivatives thereof, perylene and derivatives thereof; dyes such as polymethines, xanthenes, coumarins, cyanines and the like; metal complexes of 8-hydroxyquinoline and derivatives thereof, aromatic amines, tetraphenylcyclopentadiene and derivatives thereof, tetraphenylbutadiene and derivatives thereof, and compounds described in JP-A Nos. 57-51781 and 59-194393, and the like.

When the polymer light emitting device of the present invention contains a hole transporting layer, the hole transporting material to be used includes the above-described polymer compounds, polyvinylcarbazole and its derivatives, polysilane and its derivatives, polysiloxane derivatives having an aromatic amine on the side chain or the main chain, pyrazoline derivatives, arylamine derivatives, stilbene derivatives, triphenyldiamine derivatives, polyaniline and its derivatives, polythiophene and its derivatives, polypyrrole and its derivatives, poly(p-phenylenevinylene) and its derivatives, poly(2,5-thienylenevinylene) and its derivatives, and compounds described in JP-A Nos. 63-70257, 63-175860, 2-135359, 2-135361, 2-209988, 3-37992 and 3-152184, and the like.

Among them, preferable as the hole transporting material to be used in a hole transporting layer are high molecular weight hole transporting materials such as polyvinylcarbazole and its derivatives, polysilane and its derivatives, polysiloxane derivatives having an aromatic amine compound group on the side chain or the main chain, polyaniline and its derivatives, polythiophene and its derivatives, poly(p-phenylenevinylene) and its derivatives, poly(2,5-thienylenevinylene) and its derivatives, and the like, and more preferable are polyvinylcarbazole and its derivatives, polsilane and its derivatives, and polysiloxane derivatives having an aromatic amine on the side chain or the main chain. In the case of a low molecular weight hole transporting material, it is preferable that the hole transporting material is dispersed in a polymer binder in use.

Polyvinylcarbazole and its derivatives are obtained, for example, from a vinyl monomer by cation polymerization or radical polymerization.

As the polysilane and its derivatives, compounds described in Chemical Review (Chem. Rev.), vol. 89, p. 1359 (1989) and GB Patent No. 2300196 publication are exemplified. Also as the synthesis method, methods described in them can be used, and particularly, the Kipping method is suitably used.

In the polysiloxane derivative, the siloxane skeleton structure shows little hole transportability, thus, those having the structure of the above-mentioned low molecular weight hole transporting material in the side chain or the main chain are suitably used. Particularly, those having a hole transportable aromatic amine on the side chain or the main chain are exemplified.

As the film formation method of a hole transporting layer, a method of film formation from a mixed solution with a polymer binder is exemplified for a low molecular weight hole transporting material. In the case of a high molecular weight hole transporting material, a method of film formation from a solution is exemplified.

The solvent to be used for film formation from a solution may advantageously be one which dissolves a hole transporting material. Exemplified as the solvent are chlorine-based solvents such as chloroform, methylene chloride, dichloroethane and the like, ether solvents such as tetrahydrofuran and the like, aromatic hydrocarbon solvents such as toluene, xylene and the like, ketone solvents such as acetone, methyl ethyl ketone and the like, ester solvents such as ethyl acetate, butyl acetate, ethylcellosolve acetate and the like.

As the film formation method from a solution, there can be used application methods such as a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet print method and the like from a solution.

As the polymer binder to be mixed, those not extremely disturbing charge transportation are preferable, and those showing no strong absorption against visible light are suitably used. Exemplified as the polymer binder are polycarbonate, polyacrylate, polymethyl acrylate, polymethyl methacrylate, polystyrene, polyvinyl chloride and polysiloxane.

Regarding the film thickness of a hole transporting layer, the optimum value varies depending on the material to be used, and it may be advantageously selected so as to give suitable values of driving voltage and light emission efficiency, and a thickness at least causing no formation of pin holes is necessary, and when the film thickness is too large, the driving voltage of a device increases undesirably. Therefore, the film thickness of the hole transporting layer is usually 1 nm to 1 µm, preferably 2 nm to 500 nm, and further preferably 5 nm to 200 nm.

When the polymer light emitting device of the present invention has an electron transporting layer, the electron transporting material to be used includes the above-described polymer compounds, oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, metal complexes of 8-hydroxyquinoline and its derivatives, polyquinoline and its derivatives, polyquinoxaline and its derivatives, polyfluorene and its derivatives, and compounds described in JP-A Nos. 63-70257, 63-175860, 2-135359, 2-135361, 2-209988, 3-37992 and 3-152184.

Of them, oxadiazole derivatives, benzoquinone and its derivatives, anthraquinone and its derivatives, metal complexes of 8-hydroxyquinoline and its derivatives, polyquinoline and its derivatives, polyquinoxaline and its derivatives, polyfluorene and its derivatives are preferable, and 2-(4-biphenylyl)-5-(4-t-butylphenyl)-1,3,4-oxadiazole, benzoqinone, anthraquinone, tris(8-quinolinol)aluminum and polyquinoline are further preferable.

As the film formation method of an electron transporting layer, a vacuum vapor-deposition method from a powder and a film formation method from solution or melted state are exemplified for a low molecular weight electron transporting material, and a film formation method from solution or melted state is exemplified for a high molecular weight electron transporting material, respectively. In film formation from solution or melted state, a polymer binder may be used together.

The solvent used for film formation from a solution may advantageously be one which dissolves an electron transporting material and/or a polymer binder. Exemplified as the solvent are chlorine-based solvents such as chloroform, methylene chloride, dichloroethane and the like, ether solvents such as tetrahydrofuran and the like, aromatic hydrocarbon solvents such as toluene, xylene and the like, ketone solvents such as acetone, methyl ethyl ketone and the like, ester solvents such as ethyl acetate, butyl acetate, ethylcellosolve acetate and the like.

As the film formation method from solution or melted state, application methods such as a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet printing method and the like can be used.

As the polymer binder to be mixed, those not extremely disturbing charge transportation are preferable, and those showing no strong absorption against visible light are suitably used. Exemplified as the polymer binder are poly(N-vinylcarbazole), polyaniline and derivatives thereof, polythiophene and derivatives thereof, poly(p-phenylenevinylene) and derivatives thereof, poly(2,5-thienylenevinylene) and derivatives thereof, polycarbonate, polyacrylate, polymethyl acrylate, polymethyl methacrylate, polystyrene, polyvinyl chloride and polysiloxane.

Regarding the thickness of an electron transporting layer, the optimum value varies depending on the material to be used, and it may advantageously be selected so as to give suitable values of driving voltage and light emission efficiency, and a thickness at least causing no formation of pin holes is necessary, and when the thickness is too large, the driving voltage of a device increases undesirably. Therefore, the film thickness of the electron transporting layer is usually 1 nm to 1 µm, preferably 2 nm to 500 nm, and further preferably 5 nm to 200 nm.

Among charge transporting layers disposed adjacent to an electrode, those having a function of improving charge injection efficiency from an electrode and having an effect of lowering the driving voltage of a device are, in particularly, called generally a charge injection layer (hole injection layer, electron injection layer).

Further, for improving close adherence with an electrode or improving charge injection from an electron, the above-mentioned charge injection layer or insulation layer may be disposed adjacent to the electrode, alternatively, for improving close adherence of an interface or preventing mixing, a thin buffer layer may be inserted into an interface of a charge transporting layer and a light emitting layer.

The order and the number of layers to be laminated, and the thickness of each layer may be appropriately determined in view of light emission efficiency and device lifetime.

In the present invention, as the polymer light emitting device carrying a disposed charge injection layer (electron injection layer, hole injection layer), mentioned are polymer light emitting devices having a charge injection layer disposed adjacent to a cathode and polymer light emitting devices having a charge injection layer disposed adjacent to an anode.

Examples of the polymer light emitting device having a charge injection layer include the following devices e) to p).
e) anode/charge injection layer/light emitting layer/cathode
f) anode/light emitting layer/charge injection layer/cathode
g) anode/charge injection layer/light emitting layer/charge injection layer/cathode
h) anode/charge injection layer/hole transporting layer/light emitting layer/cathode
i) anode/hole transporting layer/light emitting layer/charge injection layer/cathode
j) anode/charge injection layer/hole transporting layer/light emitting layer/charge injection layer/cathode
k) anode/charge injection layer/light emitting layer/charge transporting layer/cathode
l) anode/light emitting layer/electron transporting layer/charge injection layer/cathode
m) anode/charge injection layer/light emitting layer/electron transporting layer/charge injection layer/cathode
n) anode/charge injection layer/hole transporting layer/light emitting layer/charge transporting layer/cathode
o) anode/hole transporting layer/light emitting layer/electron transporting layer/charge injection layer/cathode
p) anode/charge injection layer/hole transporting layer/light emitting layer/electron transporting layer/charge injection layer/cathode

As examples of the charge injection layer, exemplified are a layer containing an electric conductive polymer, a layer disposed between an anode and a hole transporting layer and containing a material having ionization potential of a value between an anode material and a hole transporting material contained in a hole transporting layer, a layer disposed between a cathode and an electron transporting layer and containing a material having electron affinity of a value between a cathode material and an electron transporting material contained in an electron transporting layer, and the like.

When the above-mentioned charge injection layer contains an electric conductive polymer, the electric conductivity of the electric conductive polymer is preferably 10⁻⁵ S/cm or more and 10³ S/cm or less, and for decreasing leak current between light emission picture elements, more preferably 10⁻⁵ S/cm or more and 10² S/cm or less, and further preferably 10⁻⁵ S/cm or more and 10¹ S/cm or less. Usually, for controlling the electric conductivity of the electric conductive polymer to 10⁻⁵ S/cm or more and 10³ S/cm or less, the electric conductive polymer is doped with a suitable amount of ions.

As the kind of ions to be doped, an anion is used when the charge injection layer is a hole injection layer and a cation is used when the charge injection layer is an electron injection layer. Examples of the anion include a polystyrenesulfonic ion, alkylbenzenesulfonic ion and camphorsulfonic ion, and examples of the cation include a lithium ion, a sodium ion, a potassium ion and a tetrabutylammonium ion.

The film thickness of the charge injection layer is, for example, 1 nm to 100 nm, preferably 2 nm to 50 nm.

The material used in the charge injection layer may be appropriately selected depending on a relation with materials of an electrode and an adjacent layer, and exemplified are the above-described polymer compounds, electric conductive polymers such as polyaniline and its derivatives, polythiophene and its derivatives, polypyrrole and its derivatives, polyphenylenevinylene and its derivatives, polythienylenevinylene and its derivatives, polyquinoline and its derivatives, polyquinoxaline and its derivatives, polymers containing an aromatic amine structure on the main chain or the side chain; and metal phthalocyanines (copper phthalocyanine and the like), and carbon.

The insulation layer has a function of making charge injection easy. The average thickness of this insulation layer is usually 0.1 to 20 nm, preferably 0.5 to 10 nm, more preferably 1 to 5 nm. As the material of the insulation layer, metal fluorides, metal oxides, organic insulating materials and the like are mentioned. As the polymer light emitting device carrying an insulation layer provided thereon, there are mentioned polymer light emitting devices in which an insulation layer is disposed adjacent to a cathode, and polymer light emitting devices in which an insulation layer is disposed adjacent to an anode.

Examples of the polymer light emitting device having an insulation layer include the following devices q) to ab).
q) anode/insulation layer/light emitting layer/cathode
r) anode/light emitting layer/insulation layer/cathode
s) anode/insulation layer/light emitting layer/insulation layer/cathode
t) anode/insulation layer/hole transporting layer/light emitting layer/cathode
u) anode/hole transporting layer/light emitting layer/insulation layer/cathode
v) anode/insulation layer/hole transporting layer/light emitting layer/insulation layer/cathode
w) anode/insulation layer/light emitting layer/electron transporting layer/cathode
x) anode/light emitting layer/electron transporting layer/insulation layer/cathode
y) anode/insulation layer/light emitting layer/electron transporting layer/insulation layer/cathode
z) anode/insulation layer/hole transporting layer/light emitting layer/electron transporting layer/cathode
aa) anode/hole transporting layer/light emitting layer/electron transporting layer/insulation layer/cathode
ab) anode/insulation layer/hole transporting layer/light emitting layer/electron transporting layer/insulation layer/cathode

The substrate which is used for forming a polymer light emitting device of the present invention may advantageously be one which does not deform in forming an electrode and forming a layer of an organic material. Examples of the substrate include substrates made of glass, plastic, polymer film, silicon and the like. In the case of an opaque substrate, it is preferable that the opposite electrode is transparent or semi-transparent.

In the present invention, it is usual that at least one of electrodes consisting of an anode and cathode is transparent or semi-transparent, and it is preferable that the anode side is transparent or semi-transparent.

As the material of the anode, an electric conductive metal oxide film, a semi-transparent metal film and the like are used. Specifically, films (NESA and the like) fabricated using electric conductive materials composed of indium oxide, zinc oxide, tin oxide, and composite thereof: indium·tin·oxide (ITO), indium·zinc·oxide and the like; gold, platinum, silver, copper and the like are used, and ITO, indium·zinc·oxide, and tin oxide are preferable. As the fabrication method, a vacuum vapor-deposition method, a sputtering method, an ion plating method, a plating method and the like are mentioned. As the anode, organic transparent electric conductive films made of polyaniline and its derivatives, polythiophene and its derivatives, and the like may be used.

The thickness of an anode can be selected in view of light transmission and electric conductivity, and it is usually 10 nm to 10 µm, preferably 20 nm to 1 µm, and further preferably 50 nm to 500 nm.

For making electric charge injection easy, a layer made of a phthalocyanine derivative, an electric conductive polymer, carbon and the like, or a layer made of a metal oxide, a metal fluoride, an organic insulation material and the like, may be provided on an anode.

As the material of a cathode, materials of small work function are preferable. As the material of a cathode, for example, metals such as lithium, sodium, potassium, rubidium, cesium, beryllium, magnesium, calcium, strontium, barium, aluminum, scandium, vanadium, zinc, yttrium, indium, cerium, samarium, europium, terbium, ytterbium and the like, alloys composed of two or more of them, or alloys made of at least one of them and at least one of gold, silver, platinum, copper, manganese, titanium, cobalt, nickel, tungsten and tin; graphite or graphite intercalation compounds and the like are used. Examples of the alloy include a magnesium-silver alloy, a magnesium-indium alloy, a magnesium-aluminum alloy, an indium-silver alloy, a lithium-aluminum alloy, a lithium-magnesium alloy, a lithium-indium alloy, a calcium-aluminum alloy and the like. The cathode may take a lamination structure composed of two or more layers.

The film thickness of a cathode can be selected in view of electric conductivity and durability, and it is usually 10 nm to 10 µm, preferably 20 nm to 1 µm, and further preferably 50 nm to 500 nm.

As the cathode fabrication method, a vacuum vapor-deposition method, a sputtering method, a lamination method of thermally press-binding a metal film, and the like are used. A layer made of an electric conductive polymer, or a layer made of a metal oxide, a metal fluoride, an organic insulation material and the like, may be provided between a cathode and an organic material layer, and after fabrication of a cathode, a protective layer for protecting the polymer light emitting device may be installed. For use of the polymer light emitting device stably for a long period of time, it is preferable to install a protective layer and/or protective cover, for protecting a device from outside.

As the protective layer, resins, metal oxides, metal fluorides, metal borides and the like can be used. As the protective cover, a glass plate, and a plastic plate having a surface which has been subjected to low water permeation treatment, and the like can be used, and a method in which the cover is pasted to a device substrate with a thermosetting resin or photocuring resin to attain sealing is suitably used. When a space is kept using a spacer, blemishing of a device can be prevented easily. If an inert gas such as nitrogen, argon and the like is filled in this space, oxidation of a cathode can be prevented, further, by placing a drying agent such as barium oxide and the like in this space, it becomes easy to suppress moisture adsorbed in a production process from imparting damages to the device. It is preferable to adopt one strategy among these methods.

The polymer light emitting device of the present invention can be used for a planar light source, and displays such as a segment display, a dot matrix display, a liquid crystal display (for example, back light, etc.) and the like.

For obtaining light emission in the form of plane using the polymer light emitting device of the present invention, it may be advantages to place a planar anode and a planar cathode so as to overlap. For obtaining light emission in the form of pattern, there are a method in which a mask having a window in the form of pattern is placed on the surface of the above-mentioned planar light emitting device, a method in which an organic material layer in non-light emitting parts is formed with extremely large thickness to give substantially no light emission, a method in which either anode or cathode, or both electrodes are formed in the form pattern. By forming a pattern by any of these methods, and placing several electrodes so that ON/OFF is independently possible, a display of segment type is obtained which can display digits, letters, simple marks and the like. Further, for providing a dot matrix device, it may be permissible that both an anode and a cathode are formed in the form of stripe, and placed so as to cross. By using a method in which several polymer compounds showing different emission colors are painted separately or a method in which a color filter or a fluorescence conversion filter is used, partial color display and multi-color display are made possible. In the case of a dot matrix device, passive driving is possible, and active driving may also be carried out in combination with TFT and the like. These displays can be used as a display of a computer, a television, a portable terminal, a cellular telephone, a car navigation, a view finder of video camera, and the like.

Further, the above-mentioned planar light emitting device is of self emitting and thin type, and can be suitably used as a planar light source for back light of a liquid crystal display, or as a planar illumination light source, an optical communication light source, an electrophotography mode printer light source, or an image sensor material. If a flexible substrate is used, it can also be used as a curved light source or display.

Next, a polymer electric field effect transistor as one embodiment of organic transistors will be described.

The polymer compound of the present invention can be suitably used as a material of a polymer electric field effect transistor, particularly, as an active layer. Regarding the structure of a polymer electric field effect transistor, it may be usually advantageous that a source electrode and a drain electrode are disposed in contact with an active layer made of a polymer, further, a gate electrode is disposed sandwiching an insulation layer in contact with the active layer.

The polymer electric field effect transistor is usually formed on a supporting substrate. The supporting substrate may advantageously be one which does not disturb the property as an electric field effect transistor, and for example, glass substrates and flexible film substrates and plastic substrates can be used.

The polymer electric field effect transistor can be produced by known methods, for example, a method described in JP-A No. 5-110069.

It is very advantageous and preferable for production to use a polymer compound soluble in an organic solvent, in forming an active layer. As the method of film formation from a solution prepared by dissolving an organic solvent-soluble polymer compound in a solvent, application methods such as a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet printing method and the like can be used.

Preferable is an encapsulated polymer electric field effect transistor obtained by fabricating a polymer electric field effect transistor, then, encapsulating this. By this, the polymer electric field effect transistor is blocked from atmospheric air, thereby, lowering of properties of the polymer electric field effect transistor can be suppressed.

As the encapsulation method, a method of covering with an ultraviolet (UV) hardening resin, a thermosetting resin, an inorganic SiONx film or the like, a method of pasting a glass plate or a film with an UV hardening resin, a thermosetting resin or the like, and other methods are mentioned. For effectively performing blocking from atmospheric air, it is preferable that processes after fabrication of a polymer electric field effect transistor until encapsulation thereof are carried out without exposing to atmospheric air (for example, in dried nitrogen atmosphere, vacuum and the like).

Next, the photoelectric conversion device will be illustrated. The photoelectric conversion device can be used for applications such as a solar battery, an optical sensor and the like. Here, a solar battery as one embodiment of the photoelectric conversion devices will be explained.

The polymer compound of the present invention can be suitably used as the material of a solar battery, particularly, as an organic semiconductor layer of a schottky barrier type device utilizing an interface between an organic semiconductor and a metal, or as an organic semiconductor layer of a pn hetero junction type device utilizing an interface between an organic semiconductor and an inorganic semiconductor or between organic semiconductors.

Further, the polymer compound of the present invention can be suitably used as an electron donating polymer or an electron accepting polymer in a bulk hetero junction type device in which the donor-acceptor contact area is increased, or as an electron donating conjugated polymer (dispersion supporting body) in a solar battery using a high molecular weight-low molecular weight composite system, for example, a bulk hetero junction type organic photoelectric conversion device in which a fullerene derivative is dispersed as an electron acceptor. If the polymer compound of the present invention is used in a solar battery, high conversion efficiency can be obtained.

With respect to the structure of the solar battery, in the case of for example a pn hetero junction type device, it is advantageous that a p type semiconductor layer is formed on an ohmic electrode, for example, on. ITO, further, an n type semiconductor layer is laminated, and an ohmic electrode is provided thereon.

The solar battery is usually formed on a supporting substrate. The supporting substrate may advantageously be one which does not disturb the property as an organic photoelectric conversion device, and for example, glass substrates and flexible film substrates and plastic substrates can be used.

The solar battery can be produced by known methods, for example, a method described in Synth. Met., 102, 982 (1999), and a method described in Science, 270, 1789 (1995).

### EXAMPLES

Examples will be shown below for illustrating the present invention further in detail, but the present invention is not limited to them.

The polystyrene-equivalent number average molecular weight and weight average molecular weight were measured by using size exclusion chromatography (SEC) (LC-10 Avp manufactured by Shimadzu Corporation, or PL-GPC2000 manufactured by Waters).

### <Analysis condition A>

In the case of measurement by LC-10Avp, a polymer compound to be measured was dissolved in tetrahydrofuran so as to give a concentration of about 0.5 wt%, and the solution was injected in an amount of 30 µL into SEC. Tetrahydrofuran was used as the mobile phase of SEC, and allowed to flow at a flow rate of 0.6 mL/min. As the column, two pieces of TSKgel Super HM-H (manufactured by Tosoh Corp.) and a piece of TSKgel Super H2000 (manufactured by Tosoh Corp.) were connected serially. A differential refractive index detector (RID-10A: manufactured by Shimadzu Corp.) was used as a detector.

### <Analysis condition B>

In the case of measurement by PL-GPC2000, a polymer compound was dissolved in o-dichlorobenzene so as to give a concentration of about 1 wt%. o-dichlorobenzene was used as the mobile phase of GPC, and allowed to flow at a flow rate of 1 mL/min at a measurement temperature of 140°C. As the column, thee pieces of PLGEL 10 µm MIXED-B (manufactured by PL Laboratory) were connected serially.

### <Example 1> (Synthesis of compound M-1)

A compound M-1 was synthesized by the following reaction.

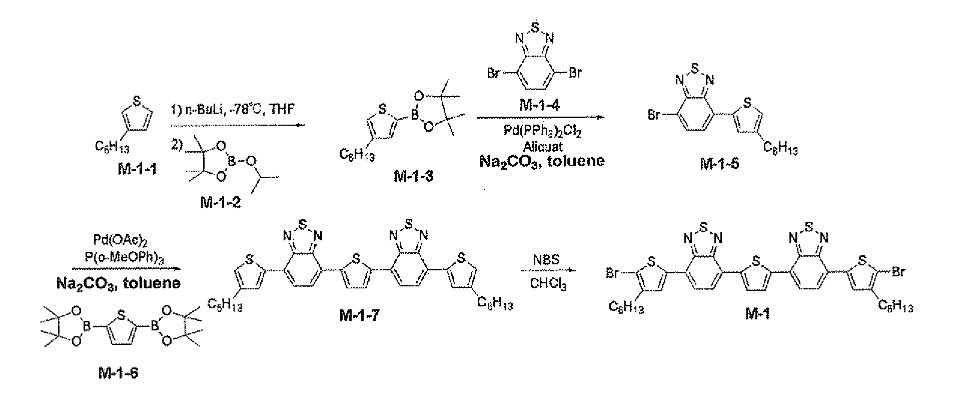

### • Synthesis of compound M-1-3

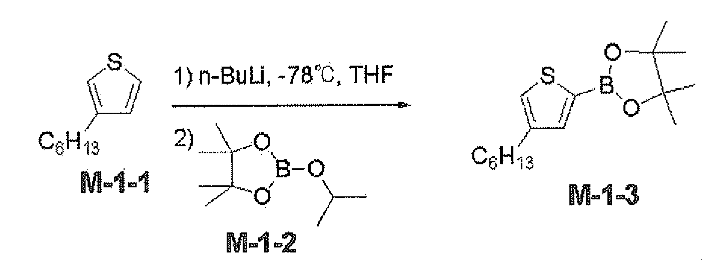

Under an inert atmosphere, into a 500 mL four-necked flask was charged a compound M-1-1 (13.46 g, 80 mmol) and 240 mL of tetrahydrofuran, and the mixture was cooled down to -78°C using acetone/dry ice, and stirred. From a dropping funnel, a n-butyllithium 1.6M hexane solution (58 mL, 88 mmol) was added, and the mixture was stirred for 1 hour, then, a compound M-1-2 (44.36 g, 240 mmol) was added and stirring of the mixture was continued at -78°C for 2 hours. Thereafter, water was added, and the tetrahydrofuran solution was concentrated, then, extracted with toluene. The extract was dried over magnesium sulfate, then, filtrated and concentrated, then, subjected to purification by a silica gel column, to obtain a compound M-1-3 (18.05 g, yield: 71%). The above-described operation was repeated twice.

### • Synthesis of compound M-1-5

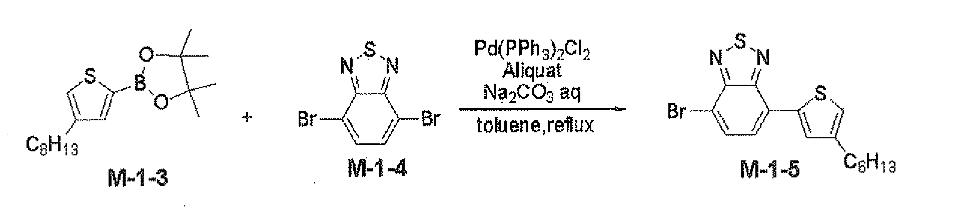

Under an inert atmosphere, into a 100 mL three-necked flask was charged the compound M-1-3 (29.4 g, 0.1 mol), a compound M-1-4 (29.3 g, 0.1 mol), trioctylmethyl ammonium chloride (18.34 g, trade name: Aliquat (registered trademark) 336, manufactured by Aldrich) and dichlorobis(triphenylphosphine)palladium (2.10 g, 3 mmol), and the mixture was dissolved in 700 mL of toluene. Thereafter, from a dropping funnel, 125 mL of a 2M sodium carbonate aqueous solution was added and the mixture was heated with stirring at 70°C for 18 hours. After completion of the reaction, the organic phase was extracted, and dried over magnesium sulfate, then, filtrated and concentrated, then, subjected to purification by a silica gel column, to obtain a compound M-1-5 (20.5 g, yield: 54%).

### • Synthesis of compound M-1-7

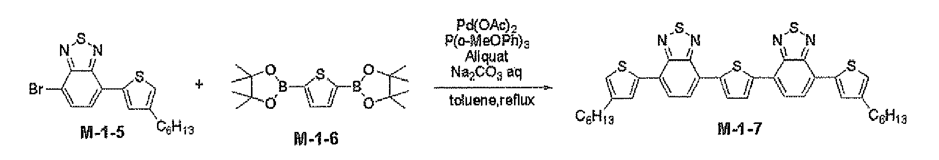

Under an inert atmosphere, into 500 mL four-necked flask was charged the compound M-1-5 (4.58 g, 12 mmol), a compound M-1-6 (2.10 g, 6 mmol), trioctylmethyl ammonium chloride (1.09 g, trade name: Aliquat (registered trademark) 336, manufactured by Aldrich), palladium acetate (68.5 mg, 0.3 mmol), tris(o-methoxyphenyl)phosphine (285 mg, 1.0 mmol) and 80 mL of toluene, and 10 mL of a 2M sodium carbonate aqueous solution was dropped while stirring, then, the mixture was heated with stirring at 105°C for 2.5 hours. The organic layer was extracted, then, washed with water, and the organic layer was dried over magnesium sulfate, then, filtrated, and the organic layer was concentrated, then, re-crystallization from chloroform was repeated, to obtain a compound M-1-7 (3.25 g, yield: 79%).

### • Synthesis of compound M-1

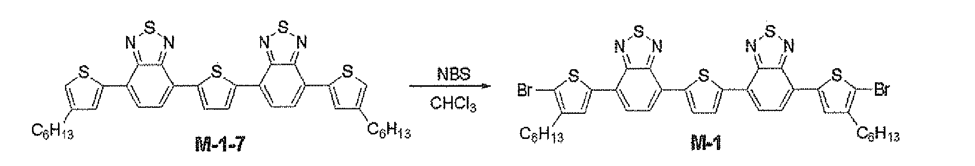

Under an inert atmosphere, into a 1000 mL four-necked flask was charged the compound M-1-7 (3.08 g, 4.5 mmol), and this was dissolved with 450 mL of chloroform. The solution was cooled down to 0°C with an ice bath, and from a dropping funnel, 30 mL of a dimethylformamide solution of N-bromosuccinimide (1.52 g, 9.0 mmol) was dropped over a period of 30 minutes. Stirring thereof was continued for 2 hours, and water was added to stop the reaction. Thereafter, the aqueous layer was removed, then, the remainder was washed with a dilute hydrochloric acid aqueous solution, saturated saline and water, and dried over magnesium sulfate, then, filtrated, and the resultant solution was concentrated. The resultant solid was subjected to re-crystallization from chloroform repeatedly, to obtain a compound M-1 (2.51 g, 66%).

### <Example 2> (Synthesis of polymer compound P-1)

### • Synthesis of compound M-2

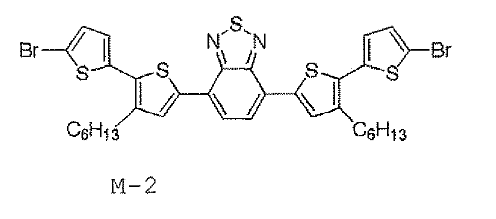

A compound M-2 was synthesized by a method described in Japanese Patent Application National Publication (Laid-Open) No. 2004-534863. That is, 4,7-bis(5-bromo-4-hexylthiophen-2-yl)-2,1,3-benzothiadiazole and 2-(tributylstannyl)thiophene were dissolved in toluene, and reacted for 18 hours while heating under reflux in the presence of tetrakis(triphenylphosphine)palladium. The reaction product was cooled down to room temperature, and filtrated through silica gel. The filtrate was concentrated and re-crystallized from hexane. The re-crystallized body was dissolved in dimethylformamide (hereinafter, referred to as "DMF"), further, a DMF solution of N-bromosuccinimide was dropped, and the mixture was stirred at room temperature overnight. The product was filtrated, and washed with methanol and deionized water. Re-crystallization from hexane was performed to obtain a compound M-2.

### • Synthesis of polymer compound P-1

2,7-bis(1,3,2-dioxaborolan-2-yl)-9,9-dioctylfluorene (1.596 g, compound A-1), 2,7-dibromo-9,9-dioctylfluorene (0.790 g, compound A-2), the compound M-2 (0.095 g), 4,7-dibromo-2,1,3-benzothiadiazole (0.176 g, compound A-3), bis(4-bromophenyl)-(4-sec-butylphenyl)-amine (0.276 g, compound A-4), the compound M-1 (0.201 g), palladium acetate (1.3 mg), tri(2-methoxyphenyl)phosphine (8.5 mg), trioctylmethyl ammonium chloride (0.39 g, trade name: Aliquat (registered trademark) 336, manufactured by Aldrich) and toluene (30 mL) were mixed, and the resultant reaction solution was heated at 105°C. Next, into the above-described reaction solution was dropped a 2M sodium carbonate aqueous solution (8.2 mL), and the mixture was refluxed for 3.5 hours, then, phenylboronic acid (39 mg), palladium acetate (1.3 mg) and tri(2-methoxyphenyl)phosphine (8.5 mg) were added, and the mixture was further refluxed for 18 hours. Then, to the above-described reaction solution was added a 1.8M sodium diethyldithiacarbamate aqueous solution (18 mL), and the mixture was stirred at 80°C for 3 hours. Thereafter, the reaction solution was cooled down to 25°C, then, washed with water (40 mL) twice, with a 3 wt% acetic acid aqueous solution (40 mL) twice and with water (40 mL) twice, and purified by passing through an alumina column and a silica gel column, to obtain a toluene solution. The resultant toluene solution was dropped into methanol (450 mL), and the mixture was stirred for 1 hour, then, the resultant solid was filtrated and dried, to obtain a polymer compound P-1. The yielded amount of the resultant polymer compound P-1 was 1.83 g. The polymer compound P-1 had a polystyrene-equivalent weight average molecular weight of 2.6×10⁵ and a polystyrene-equivalent number average molecular weight of 1.2×10⁵, under <Analysis condition A>.

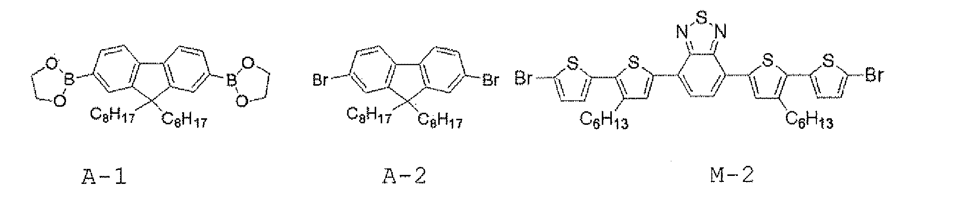

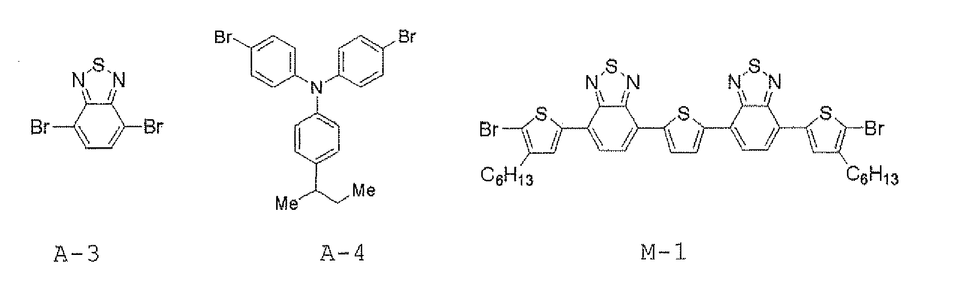

The polymer compound P-1 is a polymer compound having the following repeating units (RA-1, RA-2, RM-2, RA-3, RA-4, RM-1) at the following molar ratio (theoretical value calculated from raw materials).

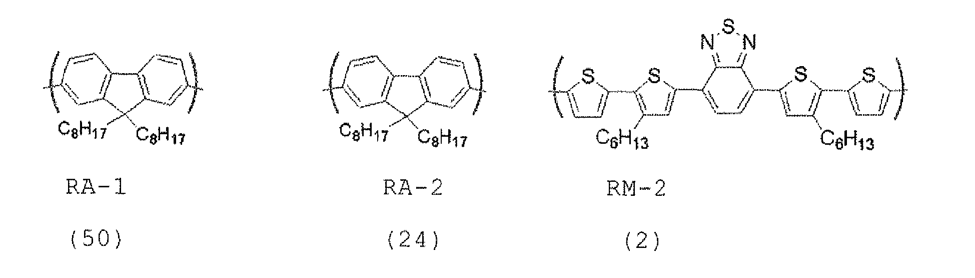

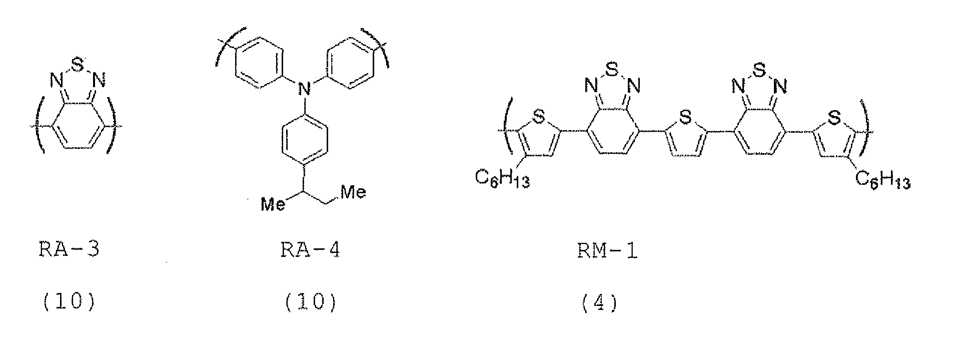

### <Comparative Example 1> (Synthesis of polymer compound P-2)

2,7-bis(1,3,2-dioxaborolan-2-yl)-9,9-dioctylfluorene (2.926 g, compound A-1), 2,7-dibromo-9,9-dioctylfluorene (1.689 g, compound A-2), the compound M-2 (0.175 g), 4,7-dibromo-2,1,3-benzothiadiazole (0.323 g, compound A-3), bis(4-bromophenyl)-(4-sec-butylphenyl)-amine (0.505 g, compound A-4), palladium acetate (2.5 mg), tri(2-methoxyphenyl)phosphine (15.5 mg), trioctylmethyl ammonium chloride (0.72 g, trade name: Aliquat (registered trademark) 336, manufactured by Aldrich) and toluene (55 mL) were mixed, and the reaction solution was heated at 105°C. Next, into the above-described reaction solution was dropped a 2M sodium carbonate aqueous solution (15 mL), and the mixture was refluxed for 2 hours, then, phenylboronic acid (71 mg), palladium acetate (2.5 mg) and tri(2-methoxyphenyl)phosphine (15.5 mg) were added, and the mixture was further refluxed for 18 hours. Then, to the above-described reaction solution was added a 1.8M sodium diethyldithiacarbamate aqueous solution (33 mL), and the mixture was stirred at 80°C for 2 hours. Thereafter, the reaction solution was cooled down to 25°C, then, washed with water (70 mL) twice, with a 3 wt% acetic acid aqueous solution (70 mL) twice and with water (70 mL) twice, and purified by passing through an alumina column and a silica gel column to obtain a toluene solution. The resultant toluene solution was dropped into methanol (850 mL), and the mixture was stirred for 1 hour, then, the resultant solid was filtrated and dried to obtain a polymer compound P-2. The yielded amount of the resultant polymer compound P-2 was 2.92 g. The polymer compound P-2 had a polystyrene-equivalent weight average molecular weight of 1.7×10⁵ and a polystyrene-equivalent number average molecular weight of 8.2×10⁴, under <Analysis condition A>.

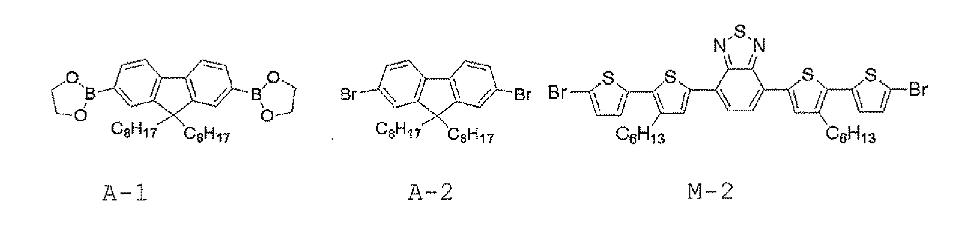

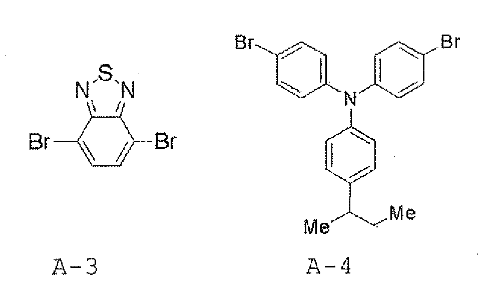

The polymer compound P-2 is a polymer compound having the following repeating units (RA-1, RA-2, RM-2, RA-3, RA-4) at the following molar ratio (theoretical value calculated from raw materials).

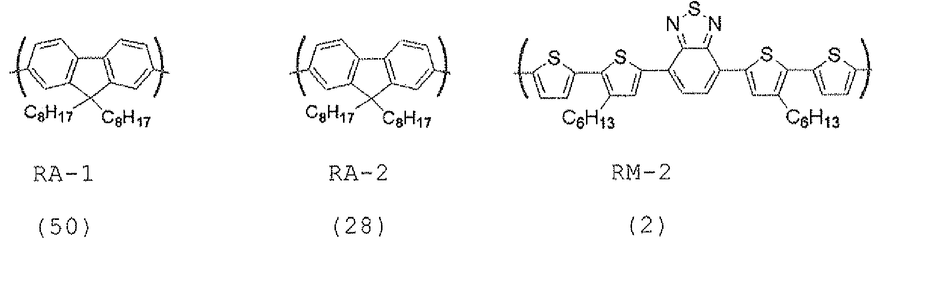

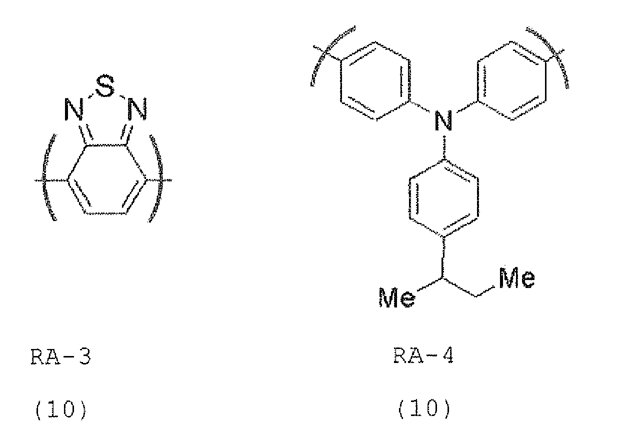

### <Synthesis Example 1> (Synthesis of polymer compound P-3)

Under an inert atmosphere, 2,7-bis(1,3,2-dioxaborolan-2-yl)-9,9-dioctylfluorene (5.20 g), bis(4-bromophenyl)-(4-sec-butylphenyl)-amine (4.50 g), palladium acetate (2.2 mg), tri(2-methylphenyl)phosphine (15.1 mg), trioctylmethyl ammonium chloride (trade name: Aliquat (registered trademark) 336, 0.91 g, manufactured by Aldrich) and toluene (70 ml) were mixed, and heated at 105°C. Into this reaction solution was dropped a 2M sodium carbonate aqueous solution (19 ml), and the mixture was refluxed for 4 hours. After the reaction, phenylboronic acid (121 mg) was added, and the mixture was further refluxed for 3 hours. Then, a sodium diethyldithiacarbamate aqueous solution was added and the mixture was stirred at 80°C for 4 hours. After cooling, the mixture was washed with water (60 ml) three times, with a 3 wt% acetic acid aqueous solution (60 ml) three times and with water (60 ml) three times, and purified by passing through an alumina column and a silica gel column. The resultant toluene solution was dropped into methanol (3 L), and stirred for 3 hours, then, the resultant solid was filtrated and dried. The yielded amount of the resultant polymer compound P-3 was 5.25 g. The polymer compound P-3 had a polystyrene-equivalent number average molecular weight of 1.2×10⁵ and a polystyrene-equivalent weight average molecular weight of 2.6×10⁵, under <Analysis condition A>.

The polymer compound P-3 is a polymer compound having the following repeating units at the following molar ratio (theoretical value calculated from raw materials).

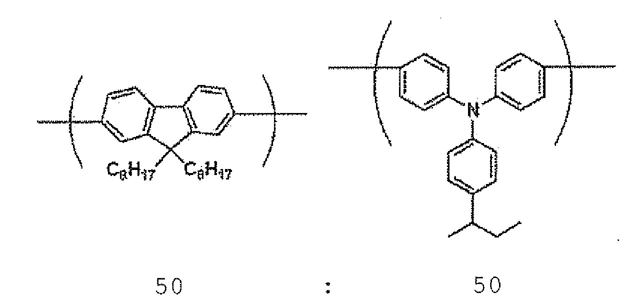

### <Example 3> (Fabrication and evaluation of polymer light emitting device)

### • Fabrication of polymer light emitting device

On a glass substrate with an ITO film having a thickness of 150 nm formed by a sputtering method, a suspension of poly(3,4)ethylenedioxythiophene/polystyrenesulfonic acid (manufactured by Bayer, trade name: BaytronP) was placed, and spin-coated to form a film having a thickness of about 50 nm, and the film was dried on a hot plate at 200°C for 10 minutes. Next, the polymer compound P-3 was dissolved at a concentration of 1.5 wt% in xylene, and the resultant xylene solution was placed on the film of BaytronP, and spin-coated to form a film, then, dried at 180°C for 60 minutes under a nitrogen atmosphere having an oxygen concentration and a moisture concentration of 10 ppm or less (by weight). This substrate was returned to room temperature under a nitrogen gas atmosphere, then, xylene was dropped on the substrate, and spin coating was performed at 2000 rpm for 30 seconds. Then, the polymer compound P-1 was dissolved at a concentration of 1.8 wt% in xylene, and the resultant xylene solution was applied by a spin coat method on the film of the polymer compound P-3, thereby forming a film having a thickness of about 120 nm. The film was dried at 130°C for 1 hour under a nitrogen atmosphere having an oxygen concentration and a moisture concentration of 10 ppm or less (by weight). After pressure reduction to 1.0×10⁻⁴ Pa or lower, barium was vapor-deposited with a thickness of about 5 nm on the film of the polymer compound P-1, then, aluminum was vapor-deposited with a thickness of about 80 nm on the layer of barium, as a cathode. After vapor deposition, encapsulation was performed using a glass substrate, to fabricate a polymer light emitting device 1. The device constitution is as described below.
ITO/Baytron P (about 50 nm)/polymer compound P-3 (20 nm)/polymer compound P-1 (about 120 nm)/Ba/Al

### • Performance of polymer light emitting device

The polymer light emitting device 1 had an emission wavelength of 713 nm, and showed an emission starting voltage of 5.21 V, and showed a voltage of 7.97 V in 1000 cd/m² light emission. Driving at constant current was carried at a current density to give an initial emission intensity of 4 mW/cm², to find a luminance 10% reduction lifetime of 0.25 hours.

### <Comparative Example 2> (Fabrication and evaluation of polymer light emitting device)

### • Fabrication of polymer light emitting device

A polymer light emitting device 2 was fabricated in the same manner as in Example 3 excepting that the polymer compound P-2 was used instead of the polymer compound P-1 in Example 3. The device constitution is as described below.
ITO/Baytron P (about 50 nm)/polymer compound P-3 (20 nm)/polymer compound P-2 (about 120 nm)/Ba/Al

### • Performance of polymer light emitting device

The polymer light emitting device 2 had an emission wavelength of 695 nm, and showed an emission starting voltage of 6.01 V, and showed a voltage of as high as 9.41 V in 1000 cd/m² light emission. Driving at constant current was carried at a current density to give an initial emission intensity of 4 mW/cm², to find a luminance 10% reduction lifetime of as short as 0.05 hours.

### <Example 4> (Synthesis of polymer compound P-4)

Into a nitrogen-purged 50 mL three-necked flask was charged 0.4272 g (0.507 mmol) of the compound M-1, 0.3008 g (0.509 mmol) of 9,9-didodecylfluorene-2,7-diboronic acid, 0.1 mg (0.001 mmol) of palladium acetate, 1.3 mg (0.004 mmol) of tri(o-methoxy)phosphine, 0.1 g of tricaprylyl methyl ammonium chloride (trade name: Aliquat (registered trademark) 336, manufactured by Aldrich) and 9 ml of toluene which had been bubbled with nitrogen previously for 30 minutes, respectively, and 0.939 ml of a sodium carbonate aqueous solution (2M/L) was dropped while heating the mixture at 105°C. After completion of dropping, the mixture was heated under reflux for 5 hours, and 11 mg of phenylboronic acid was added, and the mixture was further heated under reflux for 3 hours. Subsequently, 0.25 g of sodium N,N-diethyldithiocarbamate tri-hydrate and water were added, and the mixture was stirred at 90°C for 5 hours. Then, the aqueous layer was removed, then, the organic layer was washed with hot water three times, with 3 wt% acetic acid three times, further with hot water three times. The organic layer was dropped into methanol, and the precipitate was recovered, and dried under reduced pressure. The resultant solid was subjected to extraction by Soxhlet with acetone, then, the residual solid was recovered, and dried under reduced pressure. The dried product was re-dissolved into toluene of 100°C, and passed through a silica gel-alumina column, then, added into 300 ml of methanol. The deposited precipitate was recovered by filtration, and dried under reduced pressure. The weight of the resultant polymer compound P-4 was 0.38 g. The compound had a polystyrene-equivalent number average molecular weight of 1.23×10⁴ and a polystyrene-equivalent weight average molecular weight of 1.78×10⁴, under <Analysis condition B>.

The polymer compound P-4 is a polymer compound having the following repeating units at the following molar ratio (theoretical value calculated from raw materials).

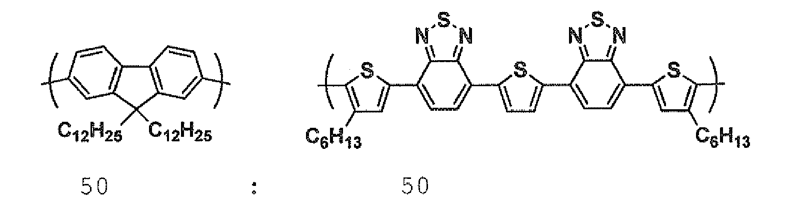

### <Example 5> (Fabrication and evaluation of organic film solar battery)

A glass substrate with an ITO film having a thickness of 150 nm formed by a sputtering method was treated by ozone UV, thereby performing a substrate treatment. Next, the polymer compound P-4 and an ortho dichlotobenzene solution of a fullerene derivative PCBM (phenyl C61-butyric acid methyl ester, manufactured by Frontier Carbon Corporation) (polymer compound P-4/PCBM = 1/3 (weight ratio)) were used and applied by spin coating to give a film thickness of about 100 nm, to fabricate a photoactive layer. Thereafter, lithium fluoride was vapor-deposited with a thickness of 4 nm by a vapor depositing machine, then, Al was vapor-deposited with a thickness of 100 nm. The shape of the resultant organic film solar battery was a regular tetragon of 2 mmx2 mm. The resultant organic film solar battery was irradiated with constant light using Solar Simulator (manufactured by BUNKOKEIKI Co., Ltd., trade name: OTENTO-SUNII: AM 1.5G filter, irradiance: 100 mW/cm²), and the generated current and voltage were measured, thereby calculating photoelectric conversion efficiency. Jsc (short-cut current density) was 3.29 mA/cm², Voc (open voltate) was 0.881 V, ff (filter factor) was 0.325, and photoelectric conversion efficiency (η) was 0.94%.

### Industrial Applicability

The polymer compound of the present invention can be suitably used as a light emitting material for a polymer light emitting device and is industrially extremely useful, since a polymer light emitting device fabricated using the polymer compound has long luminance 10% reduction lifetime.

## Claims

1. A polymer compound comprising a repeating unit represented by the formula (I): 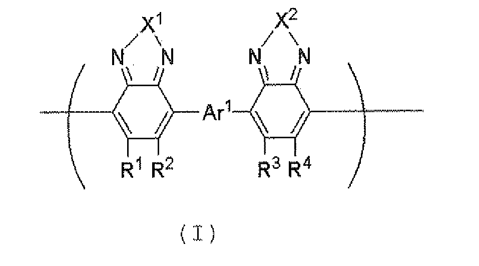 [wherein X¹ and X² are the same or mutually different and represent an oxygen atom, a sulfur atom, -N(R^{N})- or -C(R^{c1})=C(R^{c2})-, R¹, R², R³, R⁴, R^{N}, R^{c1} and R^{c2} are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and Ar¹ represents a di-valent heterocyclic group.].

2. The polymer compound according to Claim 1, comprising a repeating unit represented by the formula (II): 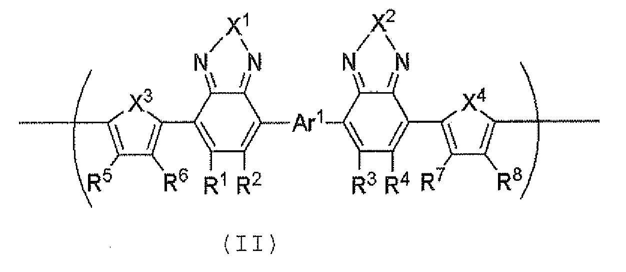 [wherein X³ and X⁴ are the same or mutually different and represent an oxygen atom, a sulfur atom or -N(R^{N})-, R⁵, R⁶, R⁷ and R⁸ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and X¹, X², R¹, R², R³, R⁴, R^{N} and Ar¹ represent the same meaning as described above.].

3. The polymer compound according to Claim 2
wherein the repeating unit represented by the formula (II) is a repeating unit represented by the formula (III): 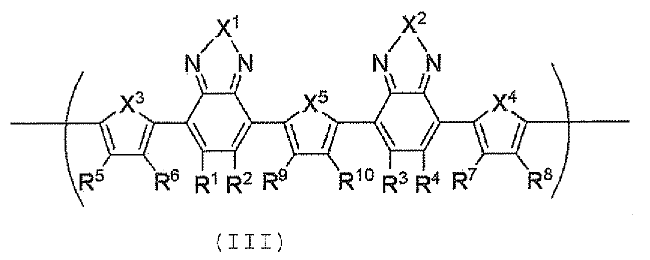 [wherein X⁵ represents an oxygen atom, a sulfur atom or -N(R^{N})-, R⁹ and R¹⁰ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and X¹, X², X³, X⁴, R¹, R², R³, R⁴, R⁵, R⁶, R⁷, R⁸ and R^{N} represent the same meaning as described above.].

4. The polymer compound according to any one of Claims 1 to 3 wherein X¹ and X² represent a sulfur atom.

5. The polymer compound according to any one of Claims 1 to 4 wherein R¹, R², R³ and R⁴ represent a hydrogen atom.

6. The polymer compound according to any one of Claims 2 to 5 wherein X³ and X⁴ represent a sulfur atom.

7. The polymer compound according to any one of Claims 3 to 6 wherein X⁵ represents a sulfur atom.

8. The polymer compound according to any one of Claims 1 to 7, further comprising a repeating unit represented by the formula (IV):
-(Ar²)- (IV)
[wherein Ar² represents an arylene group, a di-valent heterocyclic group or a di-valent aromatic amine residue.].

9. The polymer compound according to Claim 8 wherein the arylene group represented by Ar² is a group represented by the formula (V): 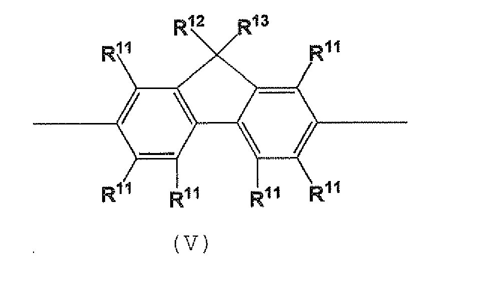 [wherein R¹² and R¹³ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an imine residue, n amide group, an acid imide group, a carboxyl group, a substituted carboxyl group, a cyano group or a nitro group. R¹¹ represents a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group. A plurality of R¹¹ may be the same or mutually different.].

10. The polymer compound according to any one of Claims 1 to 9 having a polystyrene-equivalent weight average molecular weight of 1×10³ to 1×10⁸.

11. A compound represented by the formula (VI): 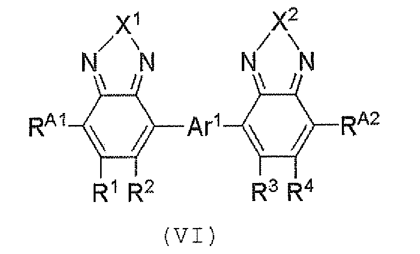 [wherein X¹ and X² are the same or mutually different and represent an oxygen atom, a sulfur atom, -N(R^{N})- or -C(R^{c1})=C(R^{c2})-, R¹, R², R³, R⁴, R^{N}, R^{A1}, R^{A2}, R^{c1} and R^{c2} are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and Ar¹ represents a di-valent heterocyclic group.].

12. The compound according to Claim 11 wherein the compound represented by the formula (VI) is a compound represented by the formula (VII). 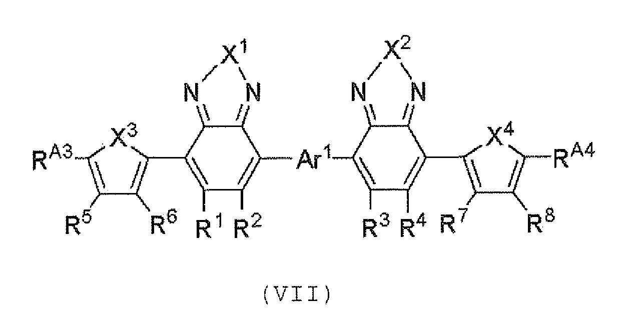 [wherein X³ and X⁴ are the same or mutually different and represent an oxygen atom, a sulfur atom or -N(R^{N})-, R^{A3}, R^{A4}, R⁵, R⁶, R⁷ and R⁸ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and X¹ X², R¹, R², R³, R⁴, R^{N} and Ar¹, represent the same meaning as described above.].

13. The compound according to Claim 12 wherein the compound represented by the formula (VII) is a compound represented by the formula (VIII): 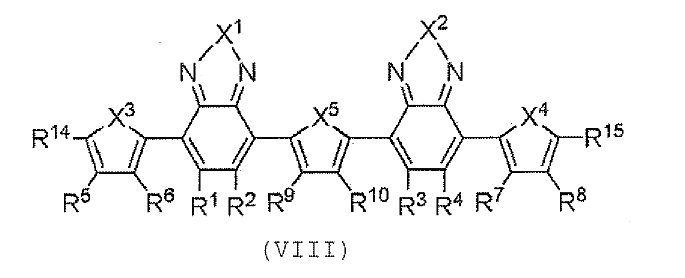 [wherein X⁵ represents an oxygen atom, a sulfur atom or -N(R^{N})-, R⁹, R¹⁰, R¹⁴ and R¹⁵ are the same or mutually different and represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a mono-valent heterocyclic group, a heterocyclic thio group, an amino group, a substituted amino group, a silyl group, a substituted silyl group, an acyl group, an acyloxy group, an imine residue, an amide group, an acid imide group, a carboxyl group, a substituted carboxyl group, a cyano group or a nitro group, and X¹ X², X³, X⁴, R¹, R², R³ ,R⁴, R⁵, R⁶, R⁷, R⁸ and R^{N} represent the same meaning as described above.].

14. The compound according to Claim 13 wherein the compound represented by the formula (VIII) is a compound represented by the formula (IX): 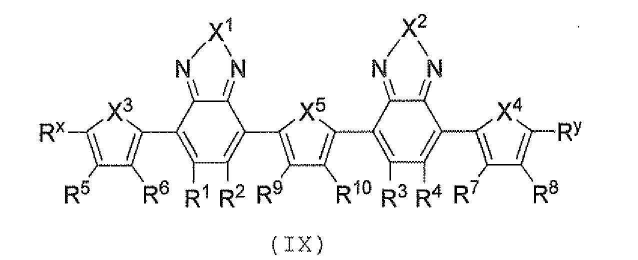 [wherein R^{x} and R^{y} are the same or mutually different and represent a halogen atom, and X¹, X², X³, X⁴, X⁵, R¹, R², R³, R⁴, R⁵, R⁶, R⁷, R⁸, R⁹, R¹⁰ and R^{N} represent the same meaning as described above.].

15. A method of producing a polymer compound having a repeating unit represented by the formula (III), comprising condensation-polymerizing a compound represented by the formula (VIII) in the presence of a transition metal compound.

16. A composition comprising the polymer compound according to any one of Claims 1 to 10.

17. A film comprising the polymer compound according to any one of Claims 1 to 10.

18. A polymer light emitting device having an anode, a cathode, and an organic layer containing the polymer compound according to any one of Claims 1 to 10 and disposed between the anode and the cathode.

19. The polymer light emitting device according to Claim 18 wherein the organic layer is a light emitting layer.

20. An organic transistor having a source electrode, a drain electrode, a gate electrode, and an organic layer containing the polymer compound according to any one of Claims 1 to 10.

21. A photoelectric conversion device having an anode, a cathode, and an organic layer containing the polymer compound according to any one of Claims 1 to 10 and disposed between the anode and the cathode.
